# EUROPEAN PATENT APPLICATION

(11) **EP 4 707 369 A1**
(43) Date of publication of application: **11.03.2026**
(21) Application number: 24223141.3
(22) Date of filing: 24.12.2024
(51) Int. Cl.: C11D 1/83, C11D 1/94, C11D 1/29, C11D 1/75

(54) **HAND DISHWASHING DETERGENT COMPOSITION**

(30) Priority: 06.09.2024 EP 24198899; 06.09.2024 EP 24198909
(71) Applicant: The Procter & Gamble Company, Cincinnati, OH 45202 (US)
(72) Inventor: BILLIAUW, Jan Julien Marie-Louise, 1853 Strombeek-Bever (BE); KEULEERS, Robby Renilde Francois, 1853 Strombeek-Bever (BE); REILMAN, Randall Thomas, Cincinnati, 45202 (US); VINSON, Phillip Kyle, Cincinnati, 45202 (US); VOLONT, Cedric Joseph, 1853 Strombeek-Bever (BE); WOS, John August, Cincinnati, 45202 (US)
(74) Representative: P&G Patent Belgium UK

(57) **Abstract**

The need for a liquid hand dishwashing composition which provides good initial sudsing while providing suds which can be readily rinsed away, is met by formulating the liquid hand dishwashing composition using a combination of anionic surfactant, comprising alkyl glycol sulfate anionic surfactant, and amphoteric/zwitterionic surfactant in a weight ratio of from 1:1 to 5:1.

## Description

### FIELD OF THE INVENTION

The invention relates to liquid hand dishwashing detergent compositions.

### BACKGROUND OF THE INVENTION

Hand dishwashing cleaning compositions have typically been formulated using a combination of anionic surfactant and amphoteric or zwitterionic co-surfactant, since the combination provides good grease removal, suds mileage in the presence of suspended greasy-particulate soil, and good low temperature stability. Grease removal and suds mileage, in particular, are seen as indicators of good, long-lasting, performance. Indeed, during manual dishwashing, the user typically relies on the level of suds to indicate the remaining cleaning efficacy of the diluted detergent composition. A high suds volume and/or stable, long-lasting suds longevity (i.e., mileage) indicates to the user that sufficient active ingredients (e.g., surfactants) remain, in order to perform the desired cleaning.

A key challenge with highly sudsing hand dishwashing detergent compositions is the need for excessive rinsing in order to remove the suds, which can be frustrating to the user. In addition, the need for additional rinsing leads to greater water usage.

Typically, alkyl ethoxylated sulfates have been used as the primary anionic surfactant in liquid hand dishwashing compositions, since they provide good sudsing, grease cleaning and low temperature stability. Alkyl ethoxylated sulfate anionic surfactants have typically been formed via the reaction of alkyl alcohols with an ethylene oxide, which gives rise to a broad distribution of degree of ethoxylation. Processes to make such alkyl ether sulfate anionic surfactants may result in trace residual amounts of 1,4-dioxane by-product being present, with the amount of dioxane formed being at least in part related to the proportion of alkyl ethoxylated sulfate anionic surfactant comprising two or more ethoxy groups. The amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps. An alternative is to use alkyl sulfate anionic surfactants which are free of ethoxylation. However, formulating with such alkyl sulfate anionic surfactants in place of alkyl ether sulfate anionic surfactant can be detrimental to suds mileage performance, low temperature stability, or both. Moreover, it has been found that formulating liquid hand dishwashing detergent compositions using alkyl sulfate anionic surfactants results in an increased need for rinsing to eliminate the suds.

As such, a need remains for a hand dishwashing composition that is more environmentally sustainable while also being high sudsing, while providing suds that are more readily rinsed away.

WO2024063990A1 relates to an aqueous light duty liquid detergent formulation including: water; a zwitterionic surfactant; and an alcohol ethoxysulfate surfactant in which 95 to 100 mol% of the alcohol ethoxysulfate surfactant has a degree of ethoxylation of 1. WO202463991A1 relates to an aqueous laundry detergent composition is provided including: water; a cleaning surfactant; wherein the cleaning surfactant comprises a blend of a nonionic surfactant and an anionic surfactant; wherein the anionic surfactant comprises an alcohol ethoxysulfate surfactant in which 95 to 100 mol% of the alcohol ethoxysulfate surfactant has a degree of ethoxylation of 1. While referred to as alcohol "ethoxysulfate" surfactants, the surfactants described in WO2024063990A1 and WO202463991A1 are not derived through processes which include an ethoxylation step, instead reacting an ethylene glycol monomer or oligomer with an alkene, before sulfation. Exemplary processes for making such "alkyl glycol" sulfates are described in WO202464645A. In contrast to alkyl ethoxylated sulfate anionic surfactants, alkyl glycol sulfate anionic surfactants are typically formed via the acid-catalysed addition of the glycol which results in the Markovnikov addition of a hydrogen (typically on the less substituted side) and an alkoxy group, resulting in the addition of an ether-bond, followed by a reaction to add SO3, i.e., a sulfation process, and subsequent neutralization to provide a counterion. This results in the alkyl glycol sulfate anionic surfactants comprising an extremely narrow distribution of ethylene glycol units in the alkyl glycol sulfate d anionic surfactant, typically from 1 to 3 ethylene glycol units per molecule of alkyl glycol sulfate anionic surfactant, or even 1 ethylene glycol unit per molecule of alkyl glycol sulfate anionic surfactant. The alkyl glycol sulfate anionic surfactant formed using such processes is also typically free of alkyl sulfate anionic surfactant that does not comprise the glycol linking group between the alkyl chain and the sulfate group. WO202464645A1 describes a process which includes the steps of contacting an olefin, an alcohol and a metallosilicate catalyst to form oligomers of an alcohol "ethoxylate" in which 95 to 100 mol% of the alcohol ethoxylate has a degree of ethoxylation of 1, and then sulphating the oligomers.

JP200610443 8A provides a liquid detergent composition providing detergency against oily stains, rinsability, handleability and handling safety, the liquid detergent composition comprises an anionic surfactant, a nonionic surfactant and water, in which the anionic surfactant comprises a secondary alkyl ether sulfate salt. JP2006137872A provides a powdery detergent composition having good fluidity, detergency and rinsing property, having little odor and excellent in disperse solubility even to low-temperature water, the powdery detergent composition comprises a higher secondary alcohol alkoxylate sulfate salt and an anionic surfactant other than the higher secondary alcohol alkoxylate sulfate salt. EP0850907A1 relates to a higher secondary alcohol alkoxylate compound composition, a method for the production thereof, and a detergent and an emulsifier using the composition.

The following journal articles describe processes whereby alkoxylate surfactants are derived from alpha-olefins: BAKKER P M: "Sulfonates and sulfates of sec-alkyl ethyl ether: detergents prepared by the addition of substituted alcohols to 1-alkenes", CHIMIE, PHYSIQUE ET APPLICATIONS PRATIQUES DES AGENTS DE SURFACE, XX, XX, 9 September 1968 (1968-09-09), pages 157 - 165, XP002075332; J. F. KNIFTON: "Detergent-range alcohol alkoxylates via vicinal glycol additions to alpha-olefins" APPLIED CATALYSIS, A, vol. 130, 1995, pages 79-88, XP002075333; P. M. BAKKER: "An exploratory study of the addition reactions of ethyleneglycol, 2-chloroethanol and 1,3-dichloro-2-propanol to 1-dodecene" JOURNAL OF THE AMERICAN OIL CHEMISTS' SOCIETY, vol. 44, September 1967, pages 517-521, XP002058548.

### SUMMARY OF THE INVENTION

The present invention relates to a liquid hand dishwashing detergent composition comprising from 5.0% to 50% by weight of the liquid hand dishwashing detergent composition of a surfactant system, wherein the surfactant system comprises: anionic surfactant, wherein the anionic surfactant comprises alkyl glycol sulfate anionic surfactant, wherein the alkyl glycol sulfate anionic surfactant has the formula:

R1CH(R2)(OCH₂CH₂)ₙOSO3⁻M⁺ (I)

wherein: R1 is independently H, alkyl, alkylene, or a mixture thereof; R2 is independently alkyl, alkylene, or a mixture thereof; the sum of the carbon atoms present in R1 and R2 is on average from 7 to 19; n is from 1 to 3, wherein 90 mol% or greater of the surfactant molecules of structure (I) have an n of 1 and 10 mol% or less of the surfactant molecules of structure (I) have an n of 2 or greater; and M⁺ is a counterion; and co-surfactant selected from the group consisting of: amphoteric co-surfactant, zwitterionic co-surfactant, and mixtures thereof; wherein the anionic surfactant and the co-surfactant are present in a weight ratio of from 1: 1 to 5: 1.

### DETAILED DESCRIPTION OF THE INVENTION

Formulating a liquid hand dishwashing detergent composition using a combination of the anionic surfactant comprising alkyl glycol sulfate anionic surfactant and amphoteric/zwitterionic surfactant, in the ratio as described herein, has been found to result in a hand dishwashing detergent composition having a desirable viscosity, good initial suds while still providing suds that are readily removed during rinsing.

Since alkyl glycol sulfate anionic surfactants are made through a process whereby ethylene glycol monomers are added to an alpha olefin, the resultant alkyl glycol sulfate anionic surfactants can be made such that essentially all of the alkyl glycol sulfate anionic surfactants comprise just one ethylene glycol unit. The extremely narrow distribution of ethylene glycol monomers in the alkyl glycol sulfate anionic surfactants of use herein, in combination with the amphoteric and/or zwitterionic surfactant at the ratios described herein, are believed to provide the improvement in viscosity, initial sudsing and rinsing of the suds.

As used herein, articles such as "a" and "an" when used in a claim, are understood to mean one or more of what is claimed or described.

The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.

The term "dishware" as used herein includes cookware and tableware made from, by non-limiting examples, ceramic, china, metal, glass, plastic (e.g., polyethylene, polypropylene, polystyrene, etc.) and wood.

The term "grease" or "greasy" as used herein means materials comprising at least in part (*i.e.,* at least 0.5 wt% by weight of the grease in the material) saturated and unsaturated fats and oils, preferably oils and fats derived from animal sources such as beef, pig and/or chicken.

The terms "include", "includes" and "including" are meant to be non-limiting.

The term "particulate soils" as used herein means inorganic and especially organic, solid soil particles, especially food particles, such as for non-limiting examples: finely divided elemental carbon, baked grease particle, and meat particles.

The term "sudsing profile" as used herein refers to the properties of a cleaning composition relating to suds character during the dishwashing process. The term "sudsing profile" of a cleaning composition includes initial suds volume generated upon dissolving and agitation, typically manual agitation, of the cleaning composition in the aqueous washing solution, and the retention of the suds during the dishwashing process. Preferably, hand dishwashing cleaning compositions characterized as having "good sudsing profile" tend to have high initial suds volume and/or sustained suds volume, particularly during a substantial portion of or for the entire manual dishwashing process. This is important as the consumer uses high suds as an indicator that enough cleaning composition has been dosed. Moreover, the consumer also uses the sustained suds volume as an indicator that enough active cleaning ingredients (e.g., surfactants) are present, even towards the end of the dishwashing process. The consumer usually renews the washing solution when the sudsing subsides. Thus, a low sudsing cleaning composition will tend to be replaced by the consumer more frequently than is necessary because of the low sudsing level.

It is understood that the test methods that are disclosed in the Test Methods Section of the present application must be used to determine the respective values of the parameters of Applicants' inventions as described and claimed herein.

All percentages are by weight of the total composition, as evident by the context, unless specifically stated otherwise. All ratios are weight ratios, unless specifically stated otherwise, and all measurements are made at 25°C, unless otherwise designated.

### Liquid cleaning composition

The cleaning composition is a liquid cleaning composition, preferably a liquid hand dishwashing cleaning composition, and hence is in liquid form. The liquid cleaning composition is preferably an aqueous cleaning composition. As such, the composition can comprise from 50% to 85%, preferably from 50% to 75%, by weight of the total composition of water.

The liquid cleaning composition has a pH of 7.0 or greater, preferably from 7.0 to 12.0, more preferably from 8.0 to 11.0, most preferably from 8.5 to 10.0, measured as a 10% aqueous solution in demineralized water at 20 degrees °C.

The liquid cleaning composition of the present invention can be Newtonian or non-Newtonian, preferably Newtonian. Preferably, the composition has a viscosity of from 10 mPa·s to 10,000 mPa·s, preferably from 100 mPa·s to 5,000 mPa·s, more preferably from 300 mPa·s to 2,000 mPa·s, or most preferably from 500 mPa·s to 1,500 mPa·s, alternatively combinations thereof.

### Surfactant System

The liquid cleaning composition comprises from 5.0% to 50%, preferably from 6.0% to 40%, most preferably from 15% to 35%, by weight of the total composition of a surfactant system.

### Anionic surfactant

The surfactant system comprises anionic surfactant. The surfactant system can comprise at least 40%, preferably from 60% to 90%, more preferably from 65% to 85% by weight of the surfactant system of the anionic surfactant. The surfactant system is preferably free of fatty acid or salt thereof, since such fatty acids impede the generation of suds.

### Alkyl glycol sulfate anionic surfactant

The surfactant system comprises an anionic surfactant, wherein the anionic surfactant comprises alkyl glycol sulfate anionic surfactant. Preferably the alkyl glycol sulfate anionic surfactant is present at a level of from 5.0 % to 50 % preferably from 10 % to 40 %, more preferably from 15 % to 30 % by weight of the anionic surfactant system.

Alkyl ethoxylated sulfate anionic surfactants are typically formed through processes whereby an alkyl alcohol is first ethoxylated, before being sulfated. In contrast, alkyl glycol sulfate anionic surfactants are typically prepared by first reacting an ethylene glycol monomer or oligomer with an alkene, before sulfation. Since the resultant sulfate surfactant is not formed using an ethoxylation step, it is technically incorrect to refer to them as alkyl ethoxylated surfactants. As such, and since they are typically formed by reacting an alkene with glycol (aliphatic diol), they are referred to here as alkyl glycol sulfate anionic surfactants, for example alkyl ethylene glycol sulfate (AEGS). Such processes, for example as described in WO202464645A, result in a much narrower distribution of the glycol groups in the resultant alkyl glycol sulfate anionic surfactants, and hence enable significant reductions in the presence and formation of dioxanes within the detergent formulae containing them. This is because little or no 1,4-dioxane is formed during the production of alkyl ethylene glycol sulfates. Moreover, by limiting the amount of alkyl ethylene glycol sulfate having two or more glycol units present, production of 1,4-dioxane through the degradation of the surfactant is substantially reduced.

The alkyl glycol sulfate anionic surfactant has the formula (I):

R1CH(R2)(OCH₂CH₂)ₙOSO₃⁻M⁺ (I)

The value of n in structure (I) has a value from 1 to 3. For example, n may be 1, 2, or 3. 90 mol% or greater of the surfactant molecules of structure (I) have an n of 1 and 10 mol% or less of the surfactant molecules of structure (I) have an n of 2 or greater. More preferably, 92 mol% or greater of the surfactant molecules of structure (I) have an n of 1 and 8 mol% or less of the surfactant molecules of structure (I) have an n of 2 or greater. Most preferably, 95 mol% or greater of the surfactant molecules of structure (I) have an n of 1 and 5 mol% or less of the surfactant molecules of structure (I) have an n of 2 or greater.

In formula (I), the group (OCH₂CH₂)ₙ can be derived from ethylene glycol (ethane-1,2-diol) or oligomers of ethylene glycol, wherein the suitable oligomers of ethylene glycol comprise up to 3 monomer units of the ethylene glycol. Preferably in formula (I), the group (OCH₂CH₂)ₙ is derived from ethylene glycol.

R1 is independently H, alkyl, alkylene, or a mixture thereof, preferably H, alkyl, or a mixture thereof, with a blend of H and alkyl being particularly preferred. Typically, the alkyl glycol sulfate anionic surfactant is a blend of alkyl glycol sulfate anionic surfactant of formula (I), in which R1 is H and alkyl, with the major part being alkyl glycol sulfate anionic surfactant of formula (I), in which R1 is alkyl, with a relatively minor part being alkyl glycol sulfate anionic surfactant of formula (I), in which R1 is H. This is because the alkyl glycol sulfate anionic surfactants of formula (I) are typically formed via an addition reaction which follows Markovnikov's rule, resulting in the most stable carbocation being formed on the more substituted carbon atom of the alkene bond due to induction and hyperconjugation. However, residual amounts of alkyl glycol sulfate anionic surfactant of formula (I) are formed in which R1 is H, since other less substituted, less stable carbocation are still formed as intermediates at some residual concentration. Preferably in at least 50% by weight, more preferably at least 60%, most preferably at least 80% by weight of the alkyl glycol sulfate of formula (I), R1 is alkyl, with the remainder being H. R1 preferably comprises from 1 to 6 carbon atoms, preferably from 1 to 3 carbon atoms. Preferably, in at least 80%, more preferably in at least 90% by mol of the alkyl glycol sulfate anionic surfactant of formula (I), R1 comprises 1 carbon atom, more preferably is methyl. As such, branching, and especially methyl-branching, at the C1 position is preferably present for at least 80%, or even at least 90% by mol of alkyl glycol sulfate anionic surfactant, wherein the C1 position is the carbon atom bound to the oxygen atom of the glycol sulfate group.

R2 is independently alkyl, alkylene, or a mixture thereof, preferably alkyl. R2 preferably comprises on average from 6 to 19 carbon atoms, preferably from 8 to 17 carbon atoms, more preferably from 9 to 15 carbon atoms, most preferably 10 to 13 carbon atoms. R2 can be linear, branched or a combination thereof, with R2 being preferably linear.

The sum of the carbon atoms present in R1 and R2 is on average from 7 to 19, preferably from 9 to 17, more preferably from 11 to 15, most preferably 11 to 13.

The alkyl glycol can have a mol average alkyl chain length of from 8 to 18, preferably from 10 to 14, most preferably from 12 to 13 carbon atoms, as defined by R1CH(R2).

For both R1 and R2, alkyl is preferred over alkylene as the presence of multiple double-bonds in the starting alkene can lead to reduced selectivity for the terminal double bond during the reaction to form the ether-bond. As such, while R1 and R2 can be independently saturated or unsaturated, saturated is preferred.

The distribution of R1 and R2 can be determined either analytically (for instance through ¹H NMR and/or ¹³C NMR) or can be determined from the starting materials used to make the alkyl glycol sulfate anionic surfactant.

M⁺ of structure (I) is a counterion, preferably M⁺ is an alkali metal counterion or ammonium, ethanolamine, isopropanolamine, triethylamine, triethanolamine, N-methyl diethanolamine, N,N-dimethyl ethanolamine, N,N-dimethyl propanolamine, and combinations thereof, more preferably Na⁺, K⁺, Mg²⁺, or ethanolamine, most preferably Na⁺. It will be understood that different surfactant molecules may have different materials for M⁺.

In formula (I), R1CH(R2) can be derived from suitable alkenes, especially linear alpha olefins (LAO). Linear alpha olefins are straight-chain terminal alkenes and hence comprise a double bond at the alpha (α-, 1- or primary) position, and a linear (unbranched) hydrocarbon chain. As such, they result in longer, less branched, alkyl groups in the resultant alkyl glycol sulfate anionic surfactant relative to when starting from mid-chain unsaturated alkenes. Suitable linear alpha olefins can be selected from the group consisting of: 1-dodecene, 1-tetradecene, 1-hexadecene, and mixtures thereof. Commercial sources of such alpha olefins can contain minor levels of other chain-length olefins, branched olefins, and vinylidene olefins.

Linear alpha olefins can be made using any known process, including via: the oligomerization of ethylene, Fischer-Tropsch synthesis, dehydration of alcohols, or thermal cracking of waxes. Alternatively, the olefin can be "bio-derived", as described in WO2011002284A or Yu, H., Wang, C., Lin, T. et al. "Direct production of olefins from syngas with ultrahigh carbon efficiency", Nat. Commun. 13, 5987 (2022). Alkyl glycol sulfate anionic surfactants are similar to alkyl ethoxylated sulfate anionic surfactants but have some key differences.

Alternatively, branched olefins can be used. The chemical and physical properties of the resultant alkyl glycol sulfate anionic surfactant can vary with the choice of linear or branched olefin, and also with the degree and type of branching. Examples of suitable branched olefins include propylene tetramer and butylene trimer (branched dodecene), as used in the manufacturing of oxo-alcohols by Exxon (Exxal 13^{™}) and BASF (Lutensol TDA^{®}).

Residual amounts of other ingredients can be present, such as secondary alcohols, secondary alcohol sulfates, unreacted or partially reacted alpha olefin, unreacted ethylene glycol, and unsulfated alkyl glycol. Such residual amounts of other ingredients are typically present at a level of less than 20%, preferably less than 10%, more preferably less than 5% by weight of the alkyl glycol sulfate anionic surfactant.

The alkyl glycol sulfate anionic surfactant can be present in a range of from 1.0 % to 25 % preferably from 3.0 % to 15 %, more preferably from 5.0 % to 10 % by weight of the composition.

Exemplary processes for making alkyl glycol sulfates of use in the present invention are described in WO202464645A. In contrast to alkyl ethoxylated sulfate anionic surfactants, alkyl glycol sulfate anionic surfactants are typically formed via the acid-catalysed addition of the glycol which results in the Markovnikov addition of a hydrogen (typically on the less substituted side) and an alkoxy group, resulting in the addition of an ether-bond, followed by a reaction to add SO3, i.e., a sulfation process, and subsequent neutralization to provide a counterion.

This results in the alkyl glycol sulfate anionic surfactants comprising an extremely narrow distribution of ethylene glycol units in the alkyl glycol sulfate anionic surfactant, typically from 1 to 3 ethylene glycol units per molecule of alkyl glycol sulfate anionic surfactant, or even 1 ethylene glycol unit per molecule of alkyl glycol sulfate anionic surfactant. The alkyl glycol sulfate anionic surfactant formed using such processes is also typically free of alkyl sulfate anionic surfactant that does not comprise the glycol linking group between the alkyl chain and the sulfate group.

When the alkyl glycol sulfate anionic surfactant is derived from the reaction of an alphaolefin with the glycol, methyl branching at the C1 position can be present for at least 90%, or even at least 95% by mol of alkyl glycol sulfate anionic surfactant, wherein the C1 position is the carbon atom bound to the oxygen atom of the glycol sulfate group.

### Alkyl sulfated anionic surfactant

The anionic surfactant preferably further comprises alkyl sulfated anionic surfactant. The anionic surfactant can comprise at least 25%, preferably from 30% to 90%, more preferably from 70% to 85% by weight of the anionic surfactant of alkyl sulfated anionic surfactant.

The anionic surfactant can comprise at least 70%, more preferably at least 85%, most preferably 100% by weight of the anionic surfactant of the alkyl sulfated anionic surfactant and the alkyl glycol sulfate anionic surfactant. In preferred compositions, the anionic surfactant consists of alkyl sulfated anionic surfactant and the alkyl glycol sulfate anionic surfactant. The alkyl sulfated anionic surfactant and the alkyl glycol sulfate anionic surfactant are preferably present at a weight ratio of from 19:1 to 1:2, preferably from 7:1 to 1:1, and most preferably from 5:1 to 2:1.

Without wishing to be bound by theory, it is believed that a mixture provides a surfactant packing which balances grease cleaning and suds mileage performance, especially in presence of greasy-particulate soils, low temperature stability and demonstrating a minimum impact on the targeted finished product viscosity.

The mol average alkyl chain length of the alkyl sulfate anionic surfactant or the alkyl alkoxy sulfate anionic surfactant can be from 8 to 18, preferably from 10 to 14, more preferably from 12 to 14, most preferably from 12 to 13 carbon atoms, in order to provide a combination of improved grease removal and enhanced speed of cleaning.

The alkyl chain of the alkyl sulfate anionic surfactant or the alkyl alkoxy sulfate anionic surfactant can have a mol fraction of C12 and C13 chains of at least 50%, preferably at least 65%, more preferably at least 80%, most preferably at least 90%. Suds mileage is particularly improved, especially in the presence of greasy soils, when the C13/C12 mol ratio of the alkyl chain is at least 57/43, preferably from 60/40 to 90/10, more preferably from 60/40 to 80/20, most preferably from 60/40 to 70/30, while not compromising suds mileage in the presence of particulate soils.

The relative molar amounts of C13 and C12 alkyl chains in the alkyl sulfate anionic surfactant or the alkyl alkoxy sulfate anionic surfactant can be derived from the carbon chain length distribution of the surfactants. The carbon chain length distributions of the alkyl chains of the alkyl sulfate and alkyl alkoxy sulfate surfactants can be obtained from the technical data sheets from the suppliers for the surfactant or constituent alkyl alcohol. Alternatively, the chain length distribution and average molecular weight of the fatty alcohols, used to make the alkyl sulfate anionic surfactant or the alkyl alkoxy sulfate anionic surfactant, can also be determined by methods known in the art. Such methods include capillary gas chromatography with flame ionization detection on medium polar capillary column, using hexane as the solvent. The chain length distribution is based on the starting alcohol and alkoxylated alcohol. As such, the alkyl sulfate anionic surfactant should be hydrolyzed back to the corresponding alkyl alcohol and alkyl alkoxylated alcohol before analysis, for instance using hydrochloric acid.

The alkyl sulfated surfactant can be alkoxylated or free of alkoxylation. When alkoxylated, the alkyl sulfated anionic surfactant can have an average degree of alkoxylation of less than 1.0, preferably less than 0.5, more preferably less than 0.25, even more preferably less than 0.1, with no alkoxylation being particularly preferred. When alkoxylated, ethoxylation is preferred. As such, the alkyl sulfated anionic surfactant can comprise less than 10% preferably less than 5% by weight of the alkyl sulfated anionic surfactant of an alkoxylated alkyl sulfate surfactant, more preferably wherein the alkyl sulfated anionic surfactant is free of an alkoxylated alkyl sulfate surfactant.

The average degree of alkoxylation is the mol average degree of alkoxylation (i.e., mol average alkoxylation degree) of all the alkyl sulfate anionic surfactant. Hence, when calculating the mol average alkoxylation degree of the alkyl sulfate anionic surfactant, the mols of non-alkoxylated sulfate anionic surfactant are included. The mols of alky glycol sulfate anionic surfactant are excluded. Mol average alkoxylation degree = (x1 * alkoxylation degree of surfactant 1 + x2 * alkoxylation degree of surfactant 2 + ....)/(x1 + x2 + ....) where x1, x2, ... are the number of moles of each alkyl (or alkoxy) sulfate anionic surfactant of the mixture and alkoxylation degree is the number of alkoxy groups in each alkyl sulfate anionic surfactant.

Preferred alkyl alkoxy sulfates are alkyl ethoxy sulfates.

Ethoxylated alkyl sulfate anionic surfactants are typically formed by first ethoxylating an alkyl alcohol to add ethylene oxide (C₂H₄O) to the alkyl alcohol, using epoxides as a starting material. The resultant alkyl ethoxylated nonionic surfactant is then sulfated to form the alkyl ethoxylated sulfate anionic surfactant.

When forming the alkyl ethoxylated nonionic surfactant, some polymerisation of the ethylene oxides occurs which results in a relatively broad distribution of degree of ethoxylation of the alkyl alcohol. The ethylene oxide used to make ethoxylated surfactants, can also give rise to the formation of 1,4-dioxanes, especially where the molecules of the alkyl ethoxylated sulfate anionic surfactant comprise at least two EO groups. This is because the ethylene oxide must first dimerise to form 1,4-dioxane. With alkyl ethoxylated sulfate anionic surfactants, without wishing to be bound by theory, through tight control of processing conditions and feedstock material compositions, both during alkoxylation especially ethoxylation and sulfation steps, the amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps. Processes to control 1,4-dioxane content within alkoxylated/ethoxylated alkyl sulfates have been described extensively in the art. Alternatively 1,4-dioxane level control within detergent formulations has also been described in the art through addition of 1,4-dioxane inhibitors to 1,4-dioxane comprising formulations, such as 5,6-dihydro-3-(4-morpholinyl)-1-[4-(2-oxo-1-piperidinyl)-phenyl]-2-(1-H)-pyridone, 3-α-hydroxy-7-oxo stereoisomer-mixtures of cholinic acid, 3-(N- methyl amino)-L-alanine, and mixtures thereof. However, it remains challenging to further reduce the dioxane level in alkyl ethoxylated surfactants.

Non-alkoxylated alkyl sulfate surfactants can be formed using naturally derived alkyl chains, such as those derived from palm oil or coconut oil. It has also been found that non-alkoxylated alkyl sulfate surfactants are more readily biodegradable by microorganisms in soil and natural waters.

The alkyl sulfated anionic surfactant preferably has an average degree of branching of less than 60%, more preferably less than 40%, even more preferably less than 20%, most preferably less than 10%. Particularly preferred, the alkyl sulfated anionic surfactant is linear. Linear alkyl chains are typically derived from renewable sources.

The alkyl sulfate anionic surfactant and the alkyl alkoxy sulfate anionic surfactant can comprise at least 5%, preferably at least 10%, most preferably at least 25%, by weight of the surfactant, of branching on the C2 position (as measured counting carbon atoms from the sulfate group for non-alkoxylated alkyl sulfate anionic surfactants and counting from the alkoxy-group furthest from the sulfate group for alkoxylated alkyl sulfate anionic surfactants). More preferably, greater than 75%, even more preferably greater than 90%, by weight of the total branched alkyl content consists of C1-C5 alkyl moiety, preferably C1-C2 alkyl moiety. It has been found that formulating the inventive compositions using alkyl sulfate surfactants or alkyl alkoxy sulfate surfactants having the aforementioned degree of branching results in improved low temperature stability. Such compositions require less solvent in order to achieve good physical stability at low temperatures. As such, the compositions can comprise lower levels of organic solvent, such as less than 5.0% by weight of the liquid composition of organic solvent, while still having improved low temperature stability. Higher surfactant branching also provides faster initial suds generation, but typically less suds mileage. The weight average branching, described herein, has been found to provide improved low temperature stability, initial foam generation and suds longevity.

The weight average degree of branching for the alkyl sulfated anionic surfactant can be calculated using the following formula. The alky glycol sulfate anionic surfactant is excluded from the calculation. Weight average degree of branching (%) = [(x1 * wt% branched alcohol 1 in alcohol 1 + x2 * wt% branched alcohol 2 in alcohol 2 + ....)/(x1 + x2 + ....)] * 100 where x1, x2, ... are the weight in grams of each alcohol in the total alcohol mixture of the alcohols which were used as starting material before (alkoxylation and) sulfation to produce the alkyl (alkoxy) sulfate anionic surfactant. In the weight average degree of branching calculation, the weight of the alkyl alcohol used to form the alkyl sulfate anionic surfactant which is not branched is included.

The weight average degree of branching and the distribution of branching can typically be obtained from the technical data sheet for the surfactant or constituent alkyl alcohol. Alternatively, the branching can also be determined through analytical methods known in the art, including capillary gas chromatography with flame ionization detection on medium polar capillary column, using hexane as the solvent. The weight average degree of branching and the distribution of branching is based on the starting alcohol used to produce the alkyl sulfate anionic surfactant.

The performance can be affected by the width of the alkoxylation distribution of the alkoxylated alkyl sulfate anionic surfactant, including grease cleaning, sudsing, low temperature stability and viscosity of the finished product. The alkoxylation distribution, including its broadness can be varied through the selection of catalyst and process conditions when making the alkoxylated alkyl sulfate anionic surfactant.

Suitable counterions include alkali metal cation, earth alkali metal cation, alkanolammonium or ammonium or substituted ammonium, but preferably sodium, since the use of alkanolammonium or ammonium or substituted ammonium can lead to discoloration of the composition.

Suitable examples of commercially available alkyl sulfated anionic surfactants include, those derived from alcohols sold under the Neodol^{®} brand-name by Shell, or the Lial^{®}, Isalchem^{®}, and Safol^{®} brand-names by Sasol, or some of the natural alcohols produced by The Procter & Gamble Chemicals company. The alcohols can be blended in order to achieve the desired mol fraction of C12 and C13 chains and the desired C13/C12 ratio, based on the relative fractions of C13 and C12 within the starting alcohols, as obtained from the technical data sheets from the suppliers or from analysis using methods known in the art.

### Additional anionic surfactant

The anionic surfactant can comprise additional anionic surfactant such as those selected from the group consisting of: alkyl (benzene) sulfonate surfactant, alkyl sulfosuccinate and dialkyl sulfosuccinate ester surfactants, and mixtures thereof.

Anionic alkyl sulfonate or sulfonic acid surfactants suitable for use herein include the acid and salt forms of alkylbenzene sulfonates, alkyl ester sulfonates, primary and secondary alkane sulfonates such as paraffin sulfonates, alfa or internal olefin sulfonates, alkyl sulfonated (poly)carboxylic acids, and mixtures thereof. Suitable anionic sulfonate or sulfonic acid surfactants include: C5-C20 alkylbenzene sulfonates, more preferably C10-C16 alkylbenzene sulfonates, more preferably C11-C13 alkylbenzene sulfonates, C5-C20 alkyl ester sulfonates especially C5-C20 methyl ester sulfonates, C6-C22 primary or secondary alkane sulfonates, C5-C20 sulfonated (poly)carboxylic acids, and any mixtures thereof, but preferably C11-C13 alkylbenzene sulfonates. The aforementioned surfactants can vary widely in their 2-phenyl isomer content. Compared with sulfonation of alpha olefins, the sulfonation of internal olefins can occur at any position since the double bond is randomly positioned, which leads to the position of hydrophilic sulfonate and hydroxyl groups of IOS in the middle of the alkyl chain, resulting in a variety of twin-tailed branching structures. Alkane sulfonates include paraffin sulfonates and other secondary alkane sulfonate (such as Hostapur SAS60 from Clariant).

Alkyl sulfosuccinate and dialkyl sulfosuccinate esters are organic compounds with the formula MO3SCH(CO2R')CH2CO2R where R and R' can be H or alkyl groups, and M is a counter-ion such as sodium (Na). Alkyl sulfosuccinate and dialkyl sulfosuccinate ester surfactants can be alkoxylated or non-alkoxylated, preferably non-alkoxylated. The surfactant system may comprise further anionic surfactant. However, the composition preferably comprises less than 30%, preferably less than 15%, more preferably less than 10% by weight of the surfactant system of further anionic surfactant. Most preferably, the surfactant system comprises no further anionic surfactant, preferably no other anionic surfactant than alkyl sulfated anionic surfactant and the alkyl glycol sulfate anionic surfactant.

### Co-surfactant

In order to improve surfactant packing after dilution and hence improve suds mileage, the surfactant system can comprise a co-surfactant which is selected from the group consisting of an amphoteric surfactant, a zwitterionic surfactant and mixtures thereof.

The anionic surfactant and the co-surfactant are present in a weight ratio of from 1:1 to 5:1, preferably from 1.5:1 to 4.5:1, more preferably from 2:1 to 4:1.

The composition preferably comprises from 0.1% to 20%, more preferably from 0.5% to 15% and especially from 2% to 10% by weight of the cleaning composition of the co-surfactant.

The surfactant system of the cleaning composition of the present invention preferably comprises up to 50%, preferably from 10% to 40%, more preferably from 15% to 35%, by weight of the surfactant system of the co-surfactant.

The co-surfactant is preferably an amphoteric surfactant, more preferably an amine oxide surfactant.

The amine oxide surfactant can be linear or branched, though linear are preferred. Suitable linear amine oxides are typically water-soluble, and characterized by the formula R1 - N(R2)(R3) O. R1 is a C8-18 alkyl, R1 is preferably is a linear alkyl chain, more preferably derived from natural, renewable resources such as coconut or palm kernel, with coconut being particularly preferred. R2 and R3 moieties are selected from the group consisting of C1-3 alkyl groups, C1-3 hydroxyalkyl groups, and mixtures thereof. For instance, R2 and R3 can be selected from the group consisting of: methyl, ethyl, propyl, isopropyl, 2-hydroxethyl, 2-hydroxypropyl and 3-hydroxypropyl, and mixtures thereof, though methyl is preferred for one or both of R2 and R3. The linear amine oxide surfactants in particular may include linear C10-C18 alkyl dimethyl amine oxides and linear C8-C12 alkoxy ethyl dihydroxy ethyl amine oxides.

Preferably, the amine oxide surfactant is selected from the group consisting of: alkyl dimethyl amine oxide, alkyl amido propyl dimethyl amine oxide, and mixtures thereof. Alkyl dimethyl amine oxides are particularly preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, and mixtures thereof. C12-C14 alkyl dimethyl amine oxide are particularly preferred.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the α carbon from the nitrogen on the alkyl moiety. This type of branching for the amine oxide is also known in the art as an internal amine oxide. The total sum of n1 and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a C1-3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:
a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and
b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl. In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

Suitable zwitterionic surfactants include betaine surfactants. Such betaine surfactants includes alkyl betaines, alkylamidobetaines, amidazoliniumbetaines, sulfobetaine (INCI Sultaines), phosphobetaines, and mixtures thereof, and preferably meets formula (I):

R¹-[CO-X(CH₂)ₙ]ₓ-N⁺(R²)(R₃)-(CH₂)ₘ-[CH(OH)-CH_{2]y}-Y⁻

Wherein in formula (I),
R1 is selected from the group consisting of: a saturated or unsaturated C6-22 alkyl residue, preferably C8-18 alkyl residue, more preferably a saturated C10-16 alkyl residue, most preferably a saturated C12-14 alkyl residue; R1 is preferably a linear alkyl chain, preferably derived from natural, renewable resources such as coconut or palm kernel, preferably coconut.
X is selected from the group consisting of: NH, NR4 wherein R4 is a C1-4 alkyl residue, O, and S,
n is an integer from 1 to 10, preferably 2 to 5, more preferably 3,
x is 0 or 1, preferably 1,
R2 and R3 are independently selected from the group consisting of: a C1-4 alkyl residue, hydroxy substituted such as a hydroxyethyl, and mixtures thereof, preferably both R2 and R3 are methyl,
m is an integer from 1 to 4, preferably 1, 2 or 3,
y is 0 or 1, and
Y is selected from the group consisting of: COO, SO3, OPO(ORS)O or P(O)(OR5)O, wherein R5 is H or a C1-4 alkyl residue.

Preferred betaines are the alkyl betaines of formula (Ia), the alkyl amido propyl betaine of formula (Ib), the sulfobetaine of formula (Ic) and the amido sulfobetaine of formula (Id):

R¹-N⁺(CH₃)₂-CH₂COO⁻ (Ia)

R¹-CO-NH-(CH₂)₃-N⁺(CH₃)₂-CH₂COO⁻ (Ib)

R¹-N⁺(CH₃)₂-CH₂CH(OH)CH₂SO₃⁻ (Ic)

R¹-CO-NH-(CH₂)₃-N⁺(CH₃)₂-CH₂CH(OH)CH₂SO₃⁻ (Id)

in which R1 has the same meaning as in formula (I). Particularly preferred are the carbobetaines [i.e. wherein Y-=COO- in formula (I)] of formulae (Ia) and (Ib), more preferred are the alkylamidobetaine of formula (Ib).

Suitable betaines can be selected from the group consisting or [designated in accordance with INCI]: capryl/capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine and/or laurylamidopropylbetaine are particularly preferred.

### Nonionic Surfactant

The surfactant system can further comprise a nonionic surfactant. Suitable nonionic surfactants include alkoxylated alcohol nonionic surfactants, alkyl polyglucoside nonionic surfactants, and mixtures thereof. Where the nonionic surfactant comprises a blend of alkyl polyglucoside and alkoxylated alcohol nonionic surfactant, the nonionic surfactant can comprise the alkyl polyglucoside and alkoxylated alcohol nonionic surfactant in a mass ratio of from 10:90 to 90: 10, preferably from 30:70 to 70:30, more preferably from 40:60 to 60:40.

The surfactant system of the composition of the present invention can further comprise from 1.0% to 50%, preferably from 1.25% to 25%, more preferably from 1.5% to 15%, most preferably from 1.5% to 5%, by weight of the surfactant system, of nonionic surfactant.

Alkoxylated alcohol nonionic surfactant:
Preferably, the alkoxylated alcohol non-ionic surfactant is a linear or branched, primary or secondary alkyl alkoxylated non-ionic surfactant, preferably an alkyl ethoxylated non-ionic surfactant, preferably comprising on average from 9 to 15, preferably from 10 to 14 carbon atoms in its alkyl chain and on average from 5 to 12, preferably from 6 to 10, most preferably from 7 to 8, units of ethylene oxide per mole of alcohol. The alkyl chain is preferably linear.

Suitable examples of commercially available alkoxylated alcohol nonionic surfactants include, those derived from alcohols sold under the Neodol^{®} brand-name by Shell, or the Lial^{®}, Isalchem^{®}, and Safol^{®} brand-names by Sasol, or some of the natural alcohols produced by The Procter & Gamble Chemicals company. The performance can be affected by the width of the alkoxylation distribution of the alkoxylated alcohol nonionic surfactant. The alkoxylation distribution, including its broadness can be varied through the selection of catalyst and process conditions when making the alkoxylated alcohol nonionic surfactant.

Alkyl polyglucoside nonionic surfactant:
Alkyl polyglucoside nonionic surfactants are typically more sudsing than other nonionic surfactants such as alkyl ethoxylated alcohols.

A combination of alkylpolyglucoside and anionic surfactant especially a mixture of alkyl sulfated and alkyl glycol sulfate anionic surfactant, has been found to improve polymerized grease removal, suds mileage performance, reduced viscosity variation with changes in the surfactant and/or system, and a more sustained Newtonian rheology.

The alkyl polyglucoside surfactant can be selected from C6-C18 alkyl polyglucoside surfactant. The alkyl polyglucoside surfactant can have a number average degree of polymerization of from 0.1 to 3.0, preferably from 1.0 to 2.0, more preferably from 1.2 to 1.6. The alkyl polyglucoside surfactant can comprise a blend of short chain alkyl polyglucoside surfactant having an alkyl chain comprising 10 carbon atoms or less, and mid to long chain alkyl polyglucoside surfactant having an alkyl chain comprising greater than 10 carbon atoms to 18 carbon atoms, preferably from 12 to 14 carbon atoms. The alkyl chain is preferably linear.

Short chain alkyl polyglucoside surfactants have a monomodal chain length distribution between C8-C10, mid to long chain alkyl polyglucoside surfactants have a monomodal chain length distribution between C10-C18, while mid chain alkyl polyglucoside surfactants have a monomodal chain length distribution between C12-C14. In contrast, C8 to C18 alkyl polyglucoside surfactants typically have a monomodal distribution of alkyl chains between C8 and C18, as with C8 to C16 and the like. As such, a combination of short chain alkyl polyglucoside surfactants with mid to long chain or mid chain alkyl polyglucoside surfactants have a broader distribution of chain lengths, or even a bimodal distribution, than non-blended C8 to C18 alkyl polyglucoside surfactants. Preferably, the weight ratio of short chain alkyl polyglucoside surfactant to long chain alkyl polyglucoside surfactant is from 1:1 to 10:1, preferably from 1.5:1 to 5:1, more preferably from 2:1 to 4:1. It has been found that a blend of such short chain alkyl polyglucoside surfactant and long chain alkyl polyglucoside surfactant results in faster dissolution of the detergent solution in water and improved initial sudsing, in combination with improved suds stability.

C8-C16 alkyl polyglucosides are commercially available from several suppliers (e.g., Simusol^{®} surfactants from Seppic Corporation; and Glucopon^{®} 600 CSUP, Glucopon^{®} 650 EC, Glucopon^{®} 600 CSUP/MB, and Glucopon^{®} 650 EC/MB, from BASF Corporation). Glucopon^{®} 215UP is a preferred short chain APG surfactant. Glucopon^{®} 600CSUP is a preferred mid to long chain APG surfactant.

In preferred compositions, the surfactant system can comprise an alkyl sulfated anionic surfactant having an average degree of branching of less than 10% and an alkyl glycol sulfate anionic surfactant, and alkyl polyglucoside nonionic surfactant.

### Further ingredients

The cleaning composition may optionally comprise a number of other adjunct ingredients such as builders (preferably citrate), chelants, conditioning polymers, other cleaning polymers, surface modifying polymers, structurants, emollients, humectants, skin rejuvenating actives, enzymes, carboxylic acids, scrubbing particles, perfumes, malodor control agents, pigments, dyes, opacifiers, pearlescent particles, inorganic cations such as alkaline earth metals such as Ca/Mg-ions, antibacterial agents, preservatives, viscosity adjusters (e.g., salt such as NaCl, and other mono-, di- and trivalent salts) and pH adjusters and buffering means (e.g. carboxylic acids such as citric acid, HCl, NaOH, KOH, alkanolamines, carbonates such as sodium carbonates, bicarbonates, sesquicarbonates, and alike).

Preferred further ingredients include those selected from: amphiphilic alkoxylated polyalkyleneimines, cyclic polyamines, triblock copolymers, hydroxypropylcellulose polymers, salt, hydrotropes, organic solvents, and mixtures thereof.

Amphiphilic alkoxylated polyalkyleneimine:
The composition of the present invention may further comprise from 0.05% to 2%, preferably from 0.07% to 1% by weight of the total composition of an amphiphilic polymer. Suitable amphiphilic polymers can be selected from the group consisting of: amphiphilic alkoxylated polyalkyleneimine and mixtures thereof. The amphiphilic alkoxylated polyalkyleneimine polymer has been found to reduce gel formation on the hard surfaces to be cleaned when the liquid composition is added directly to a cleaning implement (such as a sponge) before cleaning and consequently brought in contact with heavily greased surfaces, especially when the cleaning implement comprises a low amount to nil water such as when light pre-wetted sponges are used.

A preferred amphiphilic alkoxylated polyethyleneimine polymer has the general structure of formula (I): 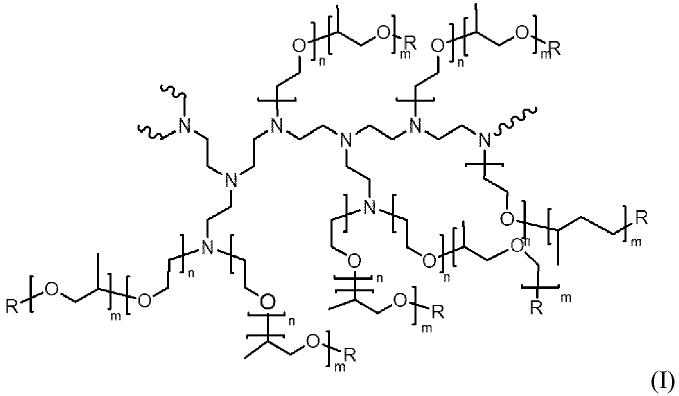 wherein the polyethyleneimine backbone has a weight average molecular weight of 600, n of formula (I) has an average of 10, m of formula (I) has an average of 7 and R of formula (I) is selected from hydrogen, a C₁-C₄ alkyl and mixtures thereof, preferably hydrogen. The degree of permanent quaternization of formula (I) may be from 0% to 22% of the polyethyleneimine backbone nitrogen atoms. The molecular weight of this amphiphilic alkoxylated polyethyleneimine polymer preferably is between 10,000 and 15,000 Da.

More preferably, the amphiphilic alkoxylated polyethyleneimine polymer has the general structure of formula (I) but wherein the polyethyleneimine backbone has a weight average molecular weight of 600 Da, n of Formula (I) has an average of 24, m of Formula (I) has an average of 16 and R of Formula (I) is selected from hydrogen, a C₁-C₄ alkyl and mixtures thereof, preferably hydrogen. The degree of permanent quaternization of Formula (I) may be from 0% to 22% of the polyethyleneimine backbone nitrogen atoms and is preferably 0%. The molecular weight of this amphiphilic alkoxylated polyethyleneimine polymer preferably is between 25,000 and 30,000, most preferably 28,000 Da.

The amphiphilic alkoxylated polyethyleneimine polymers can be made by the methods described in more detail in PCT Publication No. WO 2007/135645.

Alternatively, the compositions can be free of amphiphilic polymers.

### Cyclic Polyamine

The composition can comprise a cyclic polyamine having amine functionalities that helps cleaning. The composition of the invention preferably comprises from 0.1% to 3%, more preferably from 0.2% to 2%, and especially from 0.5% to 1%, by weight of the total composition, of the cyclic polyamine.

The cyclic polyamine has at least two primary amine functionalities. The primary amines can be in any position in the cyclic amine but it has been found that in terms of grease cleaning, better performance is obtained when the primary amines are in positions 1,3. It has also been found that cyclic amines in which one of the substituents is -CH3 and the rest are H provided for improved grease cleaning performance.

Accordingly, the most preferred cyclic polyamine for use with the cleaning composition of the present invention are cyclic polyamine selected from the group consisting of: 2-methylcyclohexane-1,3-diamine, 4-methylcyclohexane-1,3-diamine and mixtures thereof. These specific cyclic polyamines work to improve suds and grease cleaning profile through-out the dishwashing process when formulated together with the surfactant system of the composition of the present invention.

Suitable cyclic polyamines can be supplied by BASF, under the Baxxodur tradename, with Baxxodur ECX-210 being particularly preferred.

A combination of the cyclic polyamine and magnesium sulfate is particularly preferred. As such, the composition can further comprise magnesium sulfate at a level of from 0.001 % to 2.0 %, preferably from 0.005 % to 1.0 %, more preferably from 0.01 % to 0.5 % by weight of the composition.

### Triblock Copolymer

The composition of the invention can comprise a triblock copolymer. The triblock co-polymers can be present at a level of from 1% to 20%, preferably from 3% to 15%, more preferably from 5% to 12%, by weight of the total composition. Suitable triblock copolymers include alkylene oxide triblock co-polymers, defined as a triblock co-polymer having alkylene oxide moieties according to Formula (I): (EO)x(PO)y(EO)x, wherein EO represents ethylene oxide, and each x represents the number of EO units within the EO block. Each x can independently be on average of from 5 to 50, preferably from 10 to 40, more preferably from 10 to 30. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y can on average be from between 28 to 60, preferably from 30 to 55, more preferably from 30 to 48.

Preferably the triblock co-polymer has a ratio of y to each x of from 3:1 to 2:1. The triblock co-polymer preferably has a ratio of y to the average x of 2 EO blocks of from 3:1 to 2:1. Preferably the triblock co-polymer has an average weight percentage of total E-O of between 30% and 50% by weight of the tri-block co-polymer. Preferably the triblock co-polymer has an average weight percentage of total PO of between 50% and 70% by weight of the triblock co-polymer. It is understood that the average total weight % of EO and PO for the triblock co-polymer adds up to 100%. The triblock co-polymer can have an average molecular weight of between 2060 and 7880, preferably between 2620 and 6710, more preferably between 2620 and 5430, most preferably between 2800 and 4700. Average molecular weight is determined using a 1H NMR spectroscopy (*see* Thermo scientific application note No. AN52907).

Triblock co-polymers have the basic structure ABA, wherein A and B are different homopolymeric and/or monomeric units. In this case A is ethylene oxide (EO) and B is propylene oxide (PO). Those skilled in the art will recognize the phrase "block copolymers" is synonymous with this definition of "block polymers".

Triblock co-polymers according to Formula (I) with the specific EO/PO/EO arrangement and respective homopolymeric lengths have been found to enhances suds mileage performance of the liquid hand dishwashing detergent composition in the presence of greasy soils and/or suds consistency throughout dilution in the wash process.

Suitable EO-PO-EO triblock co-polymers are commercially available from BASF such as Pluronic^{®} PE series, and from the Dow Chemical Company such as Tergitol^{™} L series. Particularly preferred triblock co-polymer from BASF are sold under the tradenames Pluronic^{®} PE6400 (MW ca 2900, ca 40wt% EO) and Pluronic^{®} PE 9400 (MW ca 4600, 40 wt% EO). Particularly preferred triblock co-polymer from the Dow Chemical Company is sold under the tradename Tergitol^{™} L64 (MW ca 2700, ca 40 wt% EO).

Preferred triblock co-polymers are readily biodegradable under aerobic conditions.

### Salt:

The composition of the present invention may comprise from about 0.05% to about 2%, preferably from about 0.1% to about 1.5%, or more preferably from about 0.5% to about 1%, by weight of the total composition of a salt, preferably a monovalent or divalent inorganic salt, or a mixture thereof, more preferably selected from: sodium chloride, sodium sulfate, and mixtures thereof. Sodium chloride is most preferred.

### Hydrotrope:

The composition of the present invention may comprise from about 0.1% to about 10%, or preferably from about 0.5% to about 10%, or more preferably from about 1% to about 10% by weight of the total composition of a hydrotrope or a mixture thereof, preferably sodium cumene sulfonate.

### Organic Solvent:

The composition can comprise from about 0.1% to about 10%, or preferably from about 0.5% to about 10%, or more preferably from about 1% to about 10% by weight of the total composition of an organic solvent. Suitable organic solvents include organic solvents selected from the group consisting of: alcohols, glycols, glycol ethers, and mixtures thereof, preferably alcohols, glycols, and mixtures thereof. Ethanol is the preferred alcohol. Polyalkyleneglycols, especially polypropyleneglycol, is the preferred glycol, with polypropyleneglycols having a weight average molecular weight of from 750 Da to 1,400 Da being particularly preferred.

### Packaged product

The hand dishwashing detergent composition can be packaged in a container, typically plastic containers. Suitable containers comprise an orifice. Typically, the container comprises a cap, with the orifice typically comprised on the cap. The cap can comprise a spout, with the orifice at the exit of the spout. The spout can have a length of from 0.5 mm to 10 mm.

The orifice can have an open cross-sectional surface area at the exit of from 3 mm² to 20 mm², preferably from 3.8 mm² to 12 mm², more preferably from 5 mm² to 10 mm², wherein the container further comprises the composition according to the invention. The cross-sectional surface area is measured perpendicular to the liquid exit from the container (that is, perpendicular to the liquid flow during dispensing).

The container can typically comprise from 200 ml to 5,000 ml, preferably from 350 ml to 2000 ml, more preferably from 400 ml to 1,000 ml of the liquid hand dishwashing detergent composition.

Alternatively, the hand dishwashing detergent composition can be packaged in an inverted container. Such inverted containers typically comprise a cap at the bottom of the container, the cap comprising either a closure or a self-sealing valve, or a combination thereof. The cap preferably comprises a self-sealing valve. Suitable self-sealing valves include slit-valves. The self-sealing valve defines a dispensing orifice that is reactively openable when the pressure on the valve interior side exceeds the pressure on the valve exterior side. The bottom dispensing container can comprise an impact resistance system, such as that described in WO2019108293A1.

### Method of Washing

The invention is further directed to a method of manually washing dishware with the composition of the present invention. The method comprises the steps of delivering a composition of the present invention to a volume of water to form a wash solution and immersing the dishware in the solution. The dishware is be cleaned with the composition in the presence of water.

Optionally, the dishware can be rinsed. By "rinsing", it is meant herein contacting the dishware cleaned with the process according to the present invention with substantial quantities of appropriate solvent, typically water. By "substantial quantities", it is meant usually about 1 to about 20 L, or under running water.

The composition herein can be applied in its diluted form. Soiled dishware is contacted with an effective amount, typically from about 0.5 mL to about 20 mL (per about 25 dishes being treated), preferably from about 3 mL to about 10 mL, of the cleaning composition, preferably in liquid form, of the present invention diluted in water. The actual amount of cleaning composition used will be based on the judgment of the user and will typically depend upon factors such as the particular product formulation of the cleaning composition, including the concentration of active ingredients in the cleaning composition, the number of soiled dishes to be cleaned, the degree of soiling on the dishes, and the like. Generally, from about 0.01 mL to about 150 mL, preferably from about 3 mL to about 40 mL of a cleaning composition of the invention is combined with from about 2,000 mL to about 20,000 mL, more typically from about 5,000 mL to about 15,000 mL of water in a sink. The soiled dishware are immersed in the sink containing the diluted cleaning compositions then obtained, before contacting the soiled surface of the dishware with a cloth, sponge, or similar cleaning implement. The cloth, sponge, or similar cleaning implement may be immersed in the cleaning composition and water mixture prior to being contacted with the dishware, and is typically contacted with the dishware for a period of time ranged from about 1 to about 10 seconds, although the actual time will vary with each application and user. The contacting of cloth, sponge, or similar cleaning implement to the dishware is accompanied by a concurrent scrubbing of the dishware.

Alternatively, the composition herein can be applied in its neat form to the dish to be treated. By "in its neat form", it is meant herein that said composition is applied directly onto the surface to be treated, or onto a cleaning device or implement such as a brush, a sponge, a nonwoven material, or a woven material, without undergoing any significant dilution by the user (immediately) prior to application. "In its neat form", also includes slight dilutions, for instance, arising from the presence of water on the cleaning device, or the addition of water by the consumer to remove the remaining quantities of the composition from a bottle. Therefore, the composition in its neat form includes mixtures having the composition and water at ratios ranging from 50:50 to 100:0, preferably 70:30 to 100:0, more preferably 80:20 to 100:0, even more preferably 90:10 to 100:0 depending on the user habits and the cleaning task.

### METHODS

### pH

The pH is measured as a 10% aqueous solution in demineralized water at 20 °C, using a pH meter, such as an Orion Model 720A with an Ag/AgCl electrode (for example an Orion sure flow Electrode model 9172BN), calibrated using standardized pH 7 and pH 10 buffers.

### Viscosity measurement (Brookfield)

The viscosity is measured at 20°C with a Brookfield RT Viscometer using spindle 31 with the RPM of the viscometer adjusted to achieve a torque of between 40% and 60%, for viscosities greater than 100 mPas. For viscosities of less than 100 mPas, spindle 18 is used.

### Initial suds and foam Rinsing

Conical centrifuge tubes (50ml, supplied by Coming under the Falcon^{™} tradename) are mounted together in a placeholder rack to allow parallel measurements with matching exposure conditions.
1. 10g of test solution for each leg, consisting of a 1.0 wt% solution of the respective detergent composition in water of targeted water hardness at room temperature (20°C), is added into each tube.
2. All the tubes are simultaneously shaken 10 times in a vertical up and down direction at a speed of 2 strokes per second (1 stroke reflects a 25 cm vertical up and down motion), such that the liquid makes contact once with the screw cap during each stroke movement step, in order to generate the suds.
3. Within 30s after shaking, the initial suds volume (in ml) is determined.
4. The liquid is then decanted such that only the suds remain.
5. The centrifuge tubes containing the suds are then gently filled via a calibrated bottle dispenser (such as a Dispensette^{®} bottle dispenser from Sigma Aldrich) via the tube wall with 10mL water rinse solution of targeted water hardness at 20°C.
6. All the tubes are again simultaneously shaken.
7. Within 30s after shaking, the suds volume is re-measured and the liquid is decanted again. In this way, steps 5 through 7 represent a rinse cycle.
8. The rinse cycle is repeated until the suds volume reaches 0 ml.
9. The following data are obtained by Boltzmann fitting and the average of 3 replicates is reported: Starting suds volume - v50 (amount of cycles to reach half of the starting suds volume). A Boltzmann fitting of the curve is done by nonlinear regression: Start / ( 1 + exp (-(v50-cycle)/slope)).

### EXAMPLES

### Synthesis examples

The following blends comprising alkyl glycol sulfate and optionally alkyl sulfate anionic surfactant were prepared as follows:

### Synthesis example 1: Co-synthesis of C12 alkyl ethylene glycol sulfate and C12 alkyl sulfate blend

Co-synthesis of branched C12 alkyloxy ethanol (primarily 2-[(1-methylundecyl)oxy]ethanol (CAS 5940-87-4)) with dodecan-2-ol (CAS 10203-28-8):

To a 2-liter, single neck, round bottom reaction flask was added 50.18 g (0.298 mol) 1-dodecene, 74.08 g (1.19 mol) ethylene glycol, 49.84 g (0.262 mol) p-toluene sulfonic acid monohydrate and a magnetic stir bar. With mixing while venting to the atmosphere, the reaction flask was heated using a silicon oil bath maintained at 130°C. After 2 hours, the reaction mixture was sampled for thin layer chromatography (TLC) analysis. The TLC results indicated product formation as well as the presence of residual starting materials. The reaction flask was removed from the oil bath and allowed to cool to room temperature (21°C) at which point the reaction product was combined with 1 L aqueous solution of 10 wt% sodium carbonate, then washed three times with 2 L of ethyl acetate in a separatory funnel. The ethyl acetate layers were combined, dried by adding anhydrous sodium sulfate, filtered to remove the sodium sulfate and concentrated by evaporating solvent using rotary evaporator. The product was purified by silica gel column chromatography (80:20 hexane:ethyl acetate mobile phase), with the fractions containing the desired product being combined and concentrated by evaporation of solvent using the rotary evaporator to yield 4.012g of product. The presence of the desired 2-[(1-methylundecyl)oxy]ethanol and dodecan-2-ol products was confirmed by NMR and MS analysis.

A second synthesis and purification was completed on similar scale using the above procedure to yield 4.672 g of desired product. The two separately synthesized/purified 2-[(1-methylundecyl)oxy]ethanol and dodecan-2-ol blends were combined for subsequent sulfation.

Co-sulfation of the branched C12 alkyloxy ethanol and dodecan-2-ol blend:
A 1-L, 3-neck, round bottom flask was equipped with a magnetic stir bar, an addition funnel with pressure equalizing arm and nitrogen gas feed in the center neck, a thermometer in one side neck and a tubing vent line in the other side neck leading to a gas bubbler filled with demineralised water to trap HC1 gas evolved from reaction. A 2-Liter glass trap was positioned between the gas bubbler and the reaction flask to prevent water being drawn back into the reaction flask. 135.5 grams of the 2-[(1-methylundecyl)oxy]ethanol and dodecan-2-ol blend, and 150 ml of ACS Reagent Grade diethyl ether (EMD Millipore product # EX0190) were added to the round bottom flask. 72.6 grams (0.617 moles) of 99.0% chlorosulfonic acid (Sigma-Aldrich product # 571024) was added to the addition funnel. A nitrogen gas flow ran from the top of additional funnel, through the flask and out the side neck vent line to the gas bubbler. An ice/NaCl/water bath was placed around the reaction flask. Mixing was begun, forming a clear, dark orange solution. Once the reaction mixture reached 5 °C, the chlorosulfonic acid was dripped in at a rate that controlled the exotherm heat release and maintained the temperature at or below 10° C. The chlorosulfonic acid addition was completed within 60 minutes.

The ice/NaCl/water bath was replaced with a 22° C water bath. The vent line tube attached to the gas bubbler was switched to a vacuum tube attached to a diaphragm vacuum pump. Solvent traps cooled with a dry ice/isopropanol bath were positioned along the vacuum tube between the reaction flask and the vacuum pump to trap volatiles pulled from the reaction mixture. A dial pressure gauge (from US Gauge reading from 0-30 inches of Hg) was positioned in the vacuum tube after the solvent trap to measure vacuum pulled on system. The reaction continued to mix for 14 minutes under nitrogen gas sweep, while the vacuum system was set up.

With continued mixing, the vacuum pump is turned on to begin applying a vacuum to the reaction mixture. The vacuum level was slowly increased by incrementally decreasing the nitrogen gas flow from the addition funnel. This was done to control foaming of the reaction mixture. Eventually the nitrogen flow was completely stopped resulting in full vacuum applied to the reaction mixture (30 inches of Hg [1.02 bar] measured on the vacuum gauge indicating full vacuum applied). Full vacuum was reached 27 minutes from start of vacuum treatment. Mixing was continued under full vacuum for an additional 26 minutes at which point the reaction mixture was clear, brown in color, fluid with minimal bubbling observed, at which point the vacuum was broken with nitrogen gas flow.

With good vortex mixing using a magnetic stir bar, the reaction mixture was slowly poured into a solution of 144.8 grams (0.676 moles) of 25.2 wt% sodium methoxide in methanol reagent (Sigma-Aldrich product # 156256) diluted to a total volume of 1 Liter with ACS Reagent Grade methanol (EMD Millipore product # MX0475) contained in a glass beaker to convert the sulfated 2-[(1-methylundecyl)oxy]ethanol and dodecan-2-ol reaction product from the acid sulfate to the sodium salt. The resulting product was a cloudy mixture with a light brown precipitate with good vortex mixing. Approximately 0.2 grams of this neutralized reaction product was dissolved in approximately 0.5 grams of demineralised water and the pH was measured to be approximately 10-11 using a pH test strip. The resulting product was mixed for an additional 15 minutes, and the pH again measured to be 10-11.

The reaction product was concentrated by evaporating the solvent using a rotary evaporator with a water bath set at 50° C until mixture began to foam, at with point the mixture was poured into a glass crystallizing dish. The crystallizing dish was placed in a vacuum oven at 21° C under partial vacuum with a slow nitrogen gas flow through the oven to prevent the product mixture from foaming while further concentrating. Internal pressure of vacuum oven was approximately 4 inches of Hg [0.14 bar]. After 4 days, the concentrated reaction product was removed from the vacuum oven. The product was now a solid. The solid was broken into smaller particles using a spatula then placed into vacuum oven at 27° C under full vacuum. After 7 hours, the product was ground into smaller particles using a mortar and pestle before being placed into a vacuum oven overnight under full vacuum at 21°C. The next day, product was again ground into smaller particles using a mortar and pestle before being placed back into the vacuum oven under full vacuum at 27°C for 7 hours and then overnight under full vacuum at 21°C. The next day, the product was sampled for NMR analysis (Proton, Carbon and DEPT) and placed in vacuum oven under full vacuum at 21°C. The next day, the product was ground using a mechanical grinder, then placed into the vacuum oven under full vacuum at 27°C for 3 hours and then at 21° for 5 days. After 5 days, the product was sampled for proton NMR and standard cationic SO3 titration analysis before being transferred to a bottle for storage. 188.2 g of light brown solid product was obtained.

NMR Analysis: 0.0424 g of the product was dissolved in a mixture of 0.6 g of deuterium oxide + 0.3 g of methanol-d4 for NMR analysis (¹H, ¹³C and DEPT). By NMR, the desired target product of 2-[(1-methylundecyl)oxy]ethanol sodium sulfate and 2-dodecanol sodium sulfate was identified.

The NMR resonances for 2-dodecanol sodium sulfate were verified by the synthesis of a reference sample of 2-dodecanol sodium sulfate via the sulfation of 2-dodecanol (Sigma-Aldrich product # D221503) using the standard sulfation procedure described above.

Quantification of the level of C12 alkyl ethylene glycol sulfate and C12 alkyl sulfate in the sulfated product was determined using ¹H NMR. The level of the alkyl ethylene glycol sulfate anionic surfactant was calculated to be 86.5% by weight of the sulfated product, primarily 2-[(1-methylundecyl)oxy]ethanol sodium sulfate (i.e. where R1 is methyl and R2 is n-decyl, determined by ¹³C NMR). The level of 2-dodecanol sulfate anionic surfactant was calculated to be 13.5 % by weight of the sulfated product.

The final product was determined to be 89.6% active total sulfated surfactant blend by standard cationic SO3 titration analysis (ASTM International Standard Designation: D3049), on a solids basis. The remaining 10.4% non-surfactant solids balance were impurities such as sodium sulfate, sodium chloride and residual moisture.

### Synthesis example 2: Co-synthesis of C14 alkyl glycol sulfate and C14 alkyl sulfate blend Co-synthesis of branched C14 alkyloxy ethanol (primarily 2-[(1-methyltridecyl)oxy]ethanol (CAS# 19494-32-7)) with tetradecan-2-ol (CAS 4706-81-4):

To a 2-liter, single neck, round bottom reaction flask was added 150g (0.765 mol) 1-tetradecene, 142g (2.28 mol) ethylene glycol, 138 g (0.726 mol) p-toluene sulfonic acid monohydrate and a magnetic stir bar. With mixing while venting to the atmosphere, the reaction flask was heated using a silicone oil bath maintained at 100°C. After 72 hours, the reaction mixture was sampled for TLC analysis. TLC results indicated product formation as well as the presence of residual starting materials. The reaction flask was removed from the oil bath and allowed to cool to room temperature (21°C) at which point the reaction product was combined with 2 L aqueous solution of 10 wt% sodium carbonate, then washed three times with 1.5 L of ethyl acetate in a separatory funnel. The ethyl acetate layers were combined, dried by adding anhydrous sodium sulfate, filtered to remove sodium sulfate, then concentrated by evaporating solvent using rotary evaporator. The product was purified by silica gel column chromatography (80:20 hexane:ethyl acetate mobile phase), with the fractions containing the desired product being combined and concentrated by evaporation of solvent using rotary evaporator to yield 16 g of product. The presence of the desired products 2[(1-methyltridecyl)oxy]ethanol and tetradecan-2-ol was confirmed by NMR and MS analysis.

A second synthesis and purification was completed on similar scale using the above procedure to yield 4 g of product. The two separately synthesized/purified 2[(1-methyltridecyl)oxy]ethanol and tetradecan-2-ol blends were combined for subsequent sulfation.

Co-sulfation of the branched C14 alkyloxy ethanol and tetradecan-2-ol blend:
A 250-ml, 3-neck, round bottom flask was equipped with a magnetic stir bar, an addition funnel with pressure equalizing arm and nitrogen gas feed in the center neck, a thermometer in one side neck and a tubing vent line in the other side neck leading to a gas bubbler filled with demineralised water to trap HC1 gas evolved from reaction. A 2-Liter glass trap was positioned between the gas bubbler and the reaction flask to prevent water being drawn back into the reaction flask. 20.702 grams of the 2-[(1-methyltridecyl)oxy]ethanol and tetradecan-2-ol blend, and 85 ml of ACS Reagent Grade diethyl ether (EMD Millipore product # EX0190) were added to the round bottom flask. 9.852 grams (0.0842 moles) of 99.6% chlorosulfonic acid (Sigma-Aldrich product # 571024) was added to the addition funnel. A nitrogen gas flow ran from the top of additional funnel, through the flask and out the side neck vent line to the gas bubbler. An ice/NaCl/water bath was placed around the reaction flask. Mixing was begun, forming a clear, pale yellow solution. Once the reaction mixture reached 7 °C, the chlorosulfonic acid was dripped in at a rate that controlled the exotherm heat release and maintained the temperature at or below 10° C. The chlorosulfonic acid addition was completed within 16 minutes.

The ice/NaCl/water bath was replaced with a 22° C water bath. The vent line tube attached to the gas bubbler was switched to a vacuum tube attached to a diaphragm vacuum pump. Solvent traps cooled with a dry ice/isopropanol bath were positioned along the vacuum tube between the reaction flask and the vacuum pump to trap volatiles pulled from the reaction mixture. A dial pressure gauge (from US Gauge reading from 0-30 inches of Hg) was positioned in the vacuum tube after the solvent trap to measure vacuum pulled on system. The reaction continued to mix for 14 minutes under nitrogen gas sweep, while the vacuum system was set up.

With continued mixing, the vacuum pump is turned on to begin applying a vacuum to the reaction mixture. The vacuum level was slowly increased by incrementally decreasing the nitrogen gas flow from the addition funnel. This was done to control foaming of the reaction mixture. Eventually the nitrogen flow was completely stopped resulting in full vacuum applied to the reaction mixture (30 inches of Hg [1.02 bar] measured on the vacuum gauge indicating full vacuum applied). Full vacuum was reached 24 minutes from start of vacuum treatment. Mixing was continued under full vacuum for an additional 11 minutes at which point the reaction mixture was clear, dark orange in color, fluid with minimal bubbling observed, at which point the vacuum was broken with nitrogen gas flow.

With good vortex mixing using a magnetic stir bar, the reaction mixture was slowly poured into a solution of 19.764 grams (0.0922 moles) of 25.2 wt% sodium methoxide in methanol reagent (Sigma-Aldrich product # 156256) diluted with 85 ml of ACS Reagent Grade methanol (EMD Millipore product # MX0475) contained in a 500-ml, single neck, round bottom flask to convert the sulfated 2-[(1-methyltridecyl)oxy]ethanol and tetradecan-2-ol reaction product from the acid sulfate to the sodium salt. The resulting product was a cloudy mixture with a white precipitate with good vortex mixing. Approximately 0.2 grams of this neutralized reaction product was dissolved in approximately 0.5 grams of demineralised water and the pH was measured to be approximately 10 using a pH test strip. The resulting product was mixed for an additional 15 minutes, and the pH again measured to be 10.

The reaction product was concentrated by evaporating the solvent using a rotary evaporator with a water bath set at 50° C to obtain a soft, yellow solid. The product was placed in a vacuum oven at approximately 27° C under full vacuum for 1 hour, then allowed to go overnight under full vacuum at 21° C. The next day, the product was broken into smaller particles using a spatula then placed into vacuum oven at 27° C under full vacuum. After 7 hours, the product was ground into smaller particles using a mortar and pestle before being placed into a vacuum oven overnight under full vacuum at 21°C. The next day, product was sampled for NMR analysis then placed back into the vacuum oven under full vacuum at 21°C for 3 additional days before being transferred to a bottle for storage. 27.5 g of light brown solid product was obtained.

NMR Analysis: 0.0360 g of the product was dissolved in a mixture of 0.6 g of deuterium oxide + 0.3 g of methanol-d4 for NMR analysis (¹H, ¹³C and DEPT). By NMR, the desired target product of 2-[(1-methyltridecyl)oxy]ethanol sodium sulfate and 2-tetradecanol sodium sulfate was identified.

Quantification of the level ofC14 alkyl ethylene glycol sulfate and C14 alkyl sulfate in the sulfated product was determined using ¹H NMR. The level of the alkyl glycol sulfate anionic surfactant was calculated to be 94.8% by weight of the sulfated product, primarily 2-[(1-methyltridecyl)oxy]ethanol sodium sulfate (i.e. where R1 is methyl and R2 is n-dodecyl, determined by ¹³C NMR). The level of 2-tetradecanol sulfate anionic surfactant was calculated to be 5.2 % by weight of the sulfated product.

The final product was determined to be 90.33% active total sulfated surfactant blend by standard cationic SO3 titration analysis (ASTM International Standard Designation: D3049), on a solids basis. The remaining 9.67% non-surfactant solids balance were impurities such as sodium sulfate, sodium chloride and residual moisture.

### Synthesis example 3: Co-synthesis of C16 alkyl glycol sulfate and C16 alkyl sulfate blend Co-synthesis of branched C14 alkyloxy ethanol (primarily 2-[(1-methylpentadecyl)oxy]ethanol (CAS# 30714-96-6)) with hexadecan-2-ol (CAS 14852-31-4):

To a 2-liter, single neck, round bottom reaction flask was added 100.73 g (0.453 mol) 1-hexadecene, 112.81 g (1.82 mol) ethylene glycol, 77.80 g (0.409 mol) p-toluene sulfonic acid monohydrate and a magnetic stir bar. With mixing while venting to the atmosphere, the reaction flask was heated using a silicone oil bath maintained at 130°C. After 7 hours, the reaction mixture was sampled for TLC analysis. TLC results indicated product formation as well as the presence of residual starting materials. The reaction flask was removed from the oil bath and allowed to cool to room temperature (21°C) at which point the reaction product was combined with 2 L aqueous solution of 10 wt% sodium carbonate, then washed three times with 1.5 L of ethyl acetate in a separatory funnel. The ethyl acetate layers were combined, dried by adding anhydrous sodium sulfate, filtered to remove sodium sulfate, then concentrated by evaporating solvent using rotary evaporator. The product was purified by silica gel column chromatography (80:20 hexane:ethyl acetate mobile phase), with the fractions containing the desired product being combined and concentrated by evaporation of solvent using rotary evaporator to yield 3.91 g of product. The presence of the desired products 2-[(1-methylpentadecyl)oxy]ethanol and hexadecan-2-ol was confirmed by NMR and MS analysis.

A second synthesis and purification was completed on similar scale using the above procedure to yield 4.51 g of product. The two separately synthesized/purified 2-[(1-methylpentadecyl)oxy]ethanol and hexadecan-2-ol blends were combined for subsequent sulfation.

Co-sulfation of the branched C16 alkyloxy ethanol and hexadecan-2-ol blend:
Sulfation and neutralization were completed using the same procedure as before.

Quantification of the level of C16 alkyl ethylene glycol sulfate and C16 alkyl sulfate in the sulfated product was determined using ¹H NMR. The level of the alkyl ether sulfate anionic surfactant was calculated to be 83.6% by weight of the sulfated product, primarily 2-[(1-methylpentadecyl)oxy]ethanol sodium sulfate (i.e. where R1 is methyl and R2 is n-tetradecyl, determined by ¹³C NMR). The level of 2-hexadecanol sulfate anionic surfactant was calculated to be 16.4 % by weight of the sulfated product.

The final product was determined to be 87.5% active total sulfated surfactant blend by standard cationic SO3 titration analysis (ASTM International Standard Designation: D3049), on a solids basis. The remaining 12.5% non-surfactant solids balance were impurities such as sodium sulfate, sodium chloride and residual moisture.

### Synthesis example 4: Co-synthesis of C14 alkyl glycol sulfate and C14 alkyl sulfate blend with isolation of pure C14 alkyl glycol sulfate

Co-synthesis of branched C14 alkyloxy ethanol (primarily 2-[(1-methyltridecyl)oxy]ethanol (CAS# 19494-32-7)) with tetradecan-2-ol (CAS 4706-81-4) with isolation of pure C14 alkyl glycol sulfate:
To a 500 ml, single neck, round bottom reaction flask was added 250g (1.27 mol) 1-tetradecene, 236g (3.80 mol) ethylene glycol, 208g (1.09 mol) p-toluene sulfonic acid monohydrate and a magnetic stir bar. With mixing while venting to the atmosphere, the reaction flask was heated using a silicone oil bath maintained at 120-130°C. After 48 hours, the reaction mixture was sampled for TLC analysis. TLC results indicated product formation as well as the presence of residual starting materials. The reaction flask was removed from the oil bath and allowed to cool to room temperature (21°C) at which point the reaction product was combined with 2 L aqueous solution of 10 wt% sodium carbonate, then washed three times with 1.5 L of ethyl acetate in a separatory funnel. The ethyl acetate layers were combined, dried by adding anhydrous sodium sulfate, filtered to remove sodium sulfate, then concentrated by evaporating solvent using rotary evaporator. The product was purified by silica gel column chromatography (80:20 hexane:ethyl acetate mobile phase), with the fractions containing the desired product being combined and concentrated by evaporation of solvent using rotary evaporator to yield 25.6 g of product. The presence of the desired products 2[(1-methyltridecyl)oxy]ethanol and tetradecan-2-ol were confirmed by NMR analysis.

Two additional synthesis and purification were completed on similar scale using the above procedure to yield a total of 75.57g of product. The three separately synthesized/purified 2[(1-methyltridecyl)oxy]ethanol and tetradecan-2-ol blends were combined for subsequent sulfation.

Co-sulfation of the branched C14 alkyloxy ethanol and tetradecan-2-ol blend:
A 500-ml, 3-neck, round bottom flask was equipped with a magnetic stir bar, an addition funnel with pressure equalizing arm and nitrogen gas feed in the center neck, a thermometer in one side neck and a tubing vent line in the other side neck leading to a gas bubbler filled with demineralised water to trap HC1 gas evolved from reaction. A 2-Liter glass trap was positioned between the gas bubbler and the reaction flask to prevent water being drawn back into the reaction flask. 74.656 grams of the 2-[(1-methyltridecyl)oxy]ethanol and tetradecan-2-ol blend, and 150 ml of ACS Reagent Grade diethyl ether (EMD Millipore product # EX0190) were added to the round bottom flask. 35.5 grams (0.304 moles) of 99.6% chlorosulfonic acid (Sigma-Aldrich product # 571024) was added to the addition funnel. A nitrogen gas flow ran from the top of the additional funnel, through the flask and out the side neck vent line to the gas bubbler. An ice/NaCl/water bath was placed around the reaction flask. Mixing was begun, forming a clear, dark orange solution. Once the reaction mixture reached 5 °C, the chlorosulfonic acid was dripped in at a rate that controlled the exotherm heat release and maintained the temperature at or below 10° C. The chlorosulfonic acid addition was completed within 35 minutes.

The addition funnel was removed and replaced with a nitrogen gas feed. Continued the nitrogen gas flow through the flask to the gas bubbler. The ice/NaCl/water bath was replaced with a 22° C water bath. The vent line tube attached to the gas bubbler was switched to a vacuum tube attached to a diaphragm vacuum pump. Solvent traps cooled with a dry ice/isopropanol bath were positioned along the vacuum tube between the reaction flask and the vacuum pump to trap volatiles pulled from the reaction mixture. A dial pressure gauge (from US Gauge reading from 0-30 inches of Hg) was positioned in the vacuum tube after the solvent trap to measure vacuum pulled on system. The reaction continued to mix for 20 minutes under nitrogen gas sweep, while the vacuum system was set up.

With continued mixing, the vacuum pump is turned on to begin applying a vacuum to the reaction mixture. The vacuum level was slowly increased by incrementally decreasing the nitrogen gas flow. This was done to control foaming of the reaction mixture. Eventually the nitrogen flow was completely stopped resulting in full vacuum applied to the reaction mixture (30 inches of Hg [1.02 bar] measured on the vacuum gauge indicating full vacuum applied). Full vacuum was reached 38 minutes from start of vacuum treatment. Mixing was continued under full vacuum for an additional 10 minutes at which point the reaction mixture was clear, brown in color, fluid with minimal bubbling observed, at which point the vacuum was broken with nitrogen gas flow.

With good vortex mixing using a magnetic stir bar, the reaction mixture was slowly poured into a solution of 71.2 grams (0.331 moles) of 25.2 wt% sodium methoxide in methanol reagent (Sigma-Aldrich product # 156256) diluted with 250 ml of ACS Reagent Grade methanol (EMD Millipore product # MX0475) contained in a 500-ml glass beaker to convert the sulfated 2-[(1-methyltridecyl)oxy]ethanol and tetradecan-2-ol reaction product from the acid sulfate to the sodium salt. The resulting product was a cloudy mixture with a white precipitate with good vortex mixing. Approximately 0.2 grams of this neutralized reaction product was dissolved in approximately 0.5 grams of demineralised water and the pH was measured to be approximately 11 using a pH test strip. The resulting product was mixed for an additional 15 minutes, and the pH again measured to be 11.

The reaction product was concentrated by evaporating the solvent using a rotary evaporator with a water bath set at 50° C until mixture began to foam, at with point the mixture was poured into a glass crystallizing dish. The crystallizing dish was placed in a vacuum oven at 21° C under partial vacuum with a slow nitrogen gas flow through the oven to prevent the product mixture from foaming while further concentrating. Internal pressure of vacuum oven was approximately 2 inches of Hg [0.068 bar]. The next day, the concentrated reaction product was removed from the vacuum oven. The product was now a soft solid. The solid was broken into smaller particles using a spatula then placed into vacuum oven at 27° C under full vacuum for 6 hours, then overnight under full vacuum at 21°C. The next day, the solid product was broken into smaller particles using a spatula then placed in vacuum oven under full vacuum at 27°C for 7 hours and then overnight under full vacuum at 21°C. The next day, the product was still a soft solid at which point it was transferred to a bottle for storage. 101 g of brown, soft solid product was obtained. The product was sampled for NMR (Proton, Carbon and DEPT) and standard cationic SO3 titration analysis.

NMR Analysis: 0.0363 g of the product was dissolved in a mixture of 0.6 g of deuterium oxide + 0.3 g of methanol-d4 for NMR analysis (¹H, ¹³C and DEPT). By NMR, the desired target product of 2-[(1-methyltridecyl)oxy]ethanol sodium sulfate and 2-tetradecanol sodium sulfate was identified.

The product was determined to be 81.1% active sulfated surfactant blend by standard cationic SO3 titration analysis (ASTM International Standard Designation: D3049), on a solids basis.

### Purification of the 2-[(1-methyltridecyl)oxy]ethanol sodium sulfate and 2-tetradecanol sodium sulfate blend:

To a 1-L, single neck, round bottom flask equipped with a magnetic stir bar was added 101g of the 2-[(1-methyltridecyl)oxy]ethanol sodium sulfate and 2-tetradecanol sodium sulfate blend product and 271g of Methanol. Stoppered flask and mixed for 2.5 hours at 21°C with good vortex mixing at which point the solid product was well dispersed as fine particles. Vacuum filtered mixture through Grade 4 filter paper and washed solid filter cake twice with 50 ml of 5°C methanol. The solid filter cake was transferred to a glass crystallizing dish and placed in a vacuum oven at 21°C under full vacuum. The next day, the solid product was ground into finer particles using a mortar and pestle and placed into vacuum oven at 21°C under full vacuum. After three days, the product was removed and transferred to a bottle for storage. 46.1 g of off-white solid product was obtained. The product was sampled for NMR (Proton, Carbon and DEPT) and standard cationic SO3 titration analysis.

NMR Analysis: 0.04 g of the product was dissolved in a mixture of 0.6 g of deuterium oxide + 0.3 g of methanol-d4 for NMR analysis (¹H, ¹³C and DEPT). By NMR, the desired target product of 2-[(1-methyltridecyl)oxy]ethanol sodium sulfate was identified in high purity with all major impurities removed including full removal of 2-tetradecanol sodium sulfate.

The product was determined to be 89.76% active 2-[(1-methyltridecyl)oxy]ethanol sodium sulfate by standard cationic SO3 titration analysis (ASTM International Standard Designation: D3049), on a solids basis. The remaining 10.24% non-surfactant solids balance were impurities such as sodium sulfate, sodium chloride and residual moisture.

### Synthesis example 5: Synthesis of C16 alkyl glycol sulfate

### Synthesis of branched C16 alkyloxy ethanol (primarily 2-[(1-methylpentadecyl)oxy]ethanol (CAS# 30714-96-6)):

To a 2-liter, single neck, round bottom reaction flask was added 101.73 g (0.453 mol) 1-hexadecene, 112.80 g (1.82 mol) ethylene glycol, 70.50 g (0.371 mol) p-toluene sulfonic acid monohydrate and a magnetic stir bar. With mixing while venting to the atmosphere, the reaction flask was heated using a silicone oil bath maintained at 130°C. After 7 hours, the reaction mixture was sampled for TLC analysis. TLC results indicated product formation as well as the presence of residual starting materials. The reaction flask was removed from the oil bath and allowed to cool to room temperature (21°C) at which point the reaction product was combined with 2 L aqueous solution of 10 wt% sodium carbonate, then washed three times with 1.5 L of ethyl acetate in a separatory funnel. The ethyl acetate layers were combined, dried by adding anhydrous sodium sulfate, filtered to remove sodium sulfate, then concentrated by evaporating solvent using rotary evaporator. The product was purified by silica gel column chromatography (80:20 hexane:ethyl acetate mobile phase), with the fractions containing the desired product being combined and concentrated by evaporation of solvent using rotary evaporator to yield 4.7 g of product. The presence of the desired product 2-[(1-methylpentadecyl)oxy]ethanol was confirmed by NMR analysis.

Two additional synthesis and purification were completed on similar scale using the above procedure to yield additional product. The three separately synthesized/purified 2-[(1-methylpentadecyl)oxy]ethanol batches were combined to yield 12.1g of product for subsequent sulfation.

### Sulfation of the branched C16 alkyloxy ethanol:

To a 100-ml, single neck, round bottom flask equipped with a magnetic stir bar was added 10.046 grams (0.035065 moles) of the 2-[(1-methylpentadecyl)oxy]ethanol and 25 ml of ACS Reagent Grade diethyl ether (EMD Millipore product # EX0190). Attached to the flask an addition funnel with pressure equalizing arm that contained 4.335 grams (0.0371 moles) of 99.6% chlorosulfonic acid (Sigma-Aldrich product # 571024). A nitrogen gas line leading from a gas bubbler was attached to the top of additional funnel. An ice/NaCl/water bath was placed around the reaction flask. Mixing was begun, forming a clear, pale yellow solution. The chlorosulfonic acid was dripped in at a rate of 1 drop every 1-3 seconds. The chlorosulfonic acid addition was completed within 12 minutes at which point the reaction mixture was clear and yellow in color. 2 ml of Diethyl Ether was added to the addition funnel to rinse in residual chlorosulfonic acid and continued to mix for an additional 5 minutes. The reaction mixture was concentrated by evaporation of solvent and residual HCl using a rotary evaporator with water bath set at 25° C to form a clear, dark orange liquid.

With good vortex mixing using a magnetic stir bar, the concentrated reaction mixture was slowly poured into a solution of 8.655 grams (0.0404 moles) of 25.2 wt% sodium methoxide in methanol reagent (Sigma-Aldrich product # 156256) diluted with 50 ml of ACS Reagent Grade methanol (EMD Millipore product # MX0475) contained in a 250-ml glass beaker to convert the sulfated 2-[(1-methylpentadecyl)oxy]ethanol reaction product from the acid sulfate to the sodium salt. The resulting product was a cloudy mixture with a white precipitate with good vortex mixing. Approximately 0.2 grams of this neutralized reaction product was dissolved in approximately 0.5 grams of demineralised water and the pH was measured to be approximately 9-10 using a pH test strip. The resulting product was mixed for an additional 15 minutes, and the pH again measured to be 9-10.

The neutralized reaction product was concentrated by evaporating the solvent using a rotary evaporator with a water bath set at 50° C to obtain a soft solid. The solid was placed in a vacuum oven at 21°C under full vacuum. The next day, the solid product was broken into smaller particles using a spatula then placed into vacuum oven at 21° C under full vacuum for 7 hours, then ground into smaller particles using a mortar and pestle. The ground product was placed in vacuum oven under full vacuum at 21°C. After three days, a sample was taken for NMR (Proton, Carbon and DEPT) analysis and the product placed in vacuum oven at 21° C under full vacuum. The next day, the product was transferred to a bottle for storage. 13.1 g of brown, solid product was obtained.

NMR Analysis: 0.0347 g of the product was dissolved in a mixture of 0.6 g of deuterium oxide + 0.3 g of methanol-d4 for NMR analysis (¹H, ¹³C and DEPT). By NMR, the desired target product of 2-[(1-methylpentadecyl)oxy]ethanol sodium sulfate was identified in high purity.

The final product was determined to be 89.70% active 2-[(1-methylpentadecyl)oxy]ethanol sodium sulfate by standard cationic SO3 titration analysis (ASTM International Standard Designation: D3049), on a solids basis. The remaining 10.30% non-surfactant solids balance were impurities such as sodium sulfate, sodium chloride and residual moisture.

### Performance evaluation

The following liquid hand dishwashing compositions were prepared by simple mixing. All the examples comprised the same level of surfactant with the type of anionic surfactant, and the ratio of anionic surfactant to co-surfactant varied.

Table 1: Example 1 comprised alkyl ethylene glycol sulfate anionic surfactant comprising essentially one ethylene glycol unit per molecule of the alkyl glycol sulfate anionic surfactant present. Comparative example A comprised non-ethoxylated linear alkyl sulfate anionic surfactant in place of the alkyl ethylene glycol sulfate anionic surfactant used in inventive example 1. Comparative example B comprised a blend of non-ethoxylated linear alkyl sulfate anionic surfactant and linear alkyl ethoxylated (EOS) sulfate anionic surfactant, such that the alkyl sulfate anionic surfactant had an average degree of ethoxylation of one. Comparative example C comprised branched alkyl ethoxylated (EO1) sulfate anionic surfactant in place of the alkyl ethylene glycol sulfate anionic surfactant used in inventive example 1. The ethanol level was adjusted for all the compositions to arrive at around 1100 cps (measured using a Brookfield RT Viscometer using spindle 31@ at 20°C). The remaining ingredients were kept the same across all the compositions.

Table 2: Comparative composition D comprised the same level of surfactant as inventive example 1, but with the level of the anionic and amphoteric surfactants adjusted to provide a ratio of anionic to co-surfactant of 6:1. Comparative composition E comprised the same level of surfactant as comparative example A, but with the level of the anionic and amphoteric surfactants adjusted to provide a ratio of anionic to co-surfactant of 6:1. Comparative composition F comprised the same level of surfactant as comparative example B, but with the level of the anionic and amphoteric surfactants adjusted to provide a ratio of anionic to co-surfactant of 6:1. Comparative composition F comprised the same level of surfactant as comparative example C, but with the level of the anionic and amphoteric surfactants adjusted to provide a ratio of anionic to co-surfactant of 6:1. For compositions D to F, the level of ethanol was kept the same as comparative example A.

The viscosity, initial sudsing and suds rinsing performance of compositions of the present invention and comparative compositions was evaluated using the test methods described herein.

**Table 1: Inventive compositions and comparative compositions having a ratio of anionic to co-surfactant of 3:1:**

| | Ex 1 | Ex A* | Ex B* | Ex C* |
|---|---|---|---|---|
| C12-16 alkyl ethylene glycol sulfate¹ | 15 | - | - | - |
| C12-C16 linear alkyl sulfate | - | 15.0 | 10 | - |
| C12-C16 linear alkyl ethoxylated (EO3.0) sulfate² | - | - | 5 | - |
| C13 branched alkyl ethoxylated (EO1.0) sulfate³ | - | - | - | 15 |
| C12-14 dimethyl amine oxide⁴ | 5.0 | 5.0 | 5.0 | 5.0 |
| Ethanol | 0.62 | 2.14 | 2.05 | 0.2 |
| Polypropylene glycol (mw 2000) | 0.35 | 0.35 | 0.35 | 0.35 |
| NaCl | 0.3 | 0.3 | 0.3 | 0.3 |
| Water + misc.⁵ | to 100% | to 100% | to 100% | to 100% |
| Trimmed to pH (using 10% aqueous solution of NaOH or HCl at 20°C) | to 9.0 | to 9.0 | to 9.0 | to 9.0 |
| | | | | |
| Viscosity (Brookfield RT Viscometer using spindle 31@ at 20°C) cps | 1104 | 1022 | 1115 | 1095 |
| Initial suds level (mm) | 42.8 | 35 | 34.8 | 35.5 |
| Rinse cycles for 50% suds reduction | 1.6 | 4.9 | 4.8 | 2.9 |
| | | | | |

| | | | | |
|---|---|---|---|---|
| * Comparative ¹ 65 wt% C12 (from synthesis Example 1), 30 wt% C14 (from synthesis Example 2), and 5 wt% C16 (from synthesis Example 3) ² Tensagex^{®} EOC970, supplied by KLK Tensachem ³ 86.6% branched C13 AE1.0S, made by ethoxylating to an average of 1.0 mol ethoxylation per mol of alcohol example 1 from the examples of EP3919594A, and then sulfating the resultant ethoxylated alcohol ⁴ supplied by Procter & Gamble ⁵ Perfume, dye, preservative | | | | |

**Table 2: comparative compositions:**

| | Ex D* | Ex E* | Ex F* | Ex G* |
|---|---|---|---|---|
| C12-16 alkyl ethylene glycol sulfate¹ | 17.15 | - | - | - |
| C12-C16 linear alkyl sulfate | - | 17.15 | 11.43 | - |
| C12-C16 linear alkyl ethoxylated (EO3.0) sulfate² | - | - | 5.72 | - |
| C13 branched alkyl ethoxylated (EO1.0) sulfate³ | - | - | - | 17.15 |
| C12-14 dimethyl amine oxide⁴ | 2.85 | 2.85 | 2.85 | 2.85 |
| Ethanol | 2.14 | 2.14 | 2.14 | 2.14 |
| Polypropylene glycol (mw 2000) | 0.35 | 0.35 | 0.35 | 0.35 |
| NaCl | 0.3 | 0.3 | 0.3 | 0.3 |
| Water + misc.⁵ | to 100% | to 100% | to 100% | to 100% |
| Trimmed to pH (using 10% aqueous solution of NaOH or HCl at 20°C) | to 9.0 | to 9.0 | to 9.0 | to 9.0 |
| | | | | |
| Viscosity (Brookfield RT Viscometer using spindle 31@ at 20°C) cps | 136 | 142 | 626 | 795 |
| Initial suds level (mm) | 39.2 | 41.2 | 41.7 | 41.6 |
| Rinse cycles for 50% suds reduction | 2.3 | 3.3 | 2.8 | 2.3 |
| | | | | |

Less ethanol had to be added in the inventive example 1 in order to reduce the viscosity to the desired level in comparison to comparative composition A which comprised non-ethoxylated linear alkyl sulfate anionic surfactant or comparative example B which comprised linear alkyl ethoxylated sulfate anionic surfactant. In addition, it can be seen from comparing the initial suds level and rinse cycles for a 50% reduction in suds, that inventive example 1, comprising the alkyl ethylene glycol sulfate anionic surfactant, provided both improved initial sudsing and improved suds rinsing, in comparison to equivalent compositions comprising non-ethoxylated linear alkyl sulfate anionic surfactant (comparative example A) or comprising linear alkyl ethoxylated sulfate anionic surfactant (comparative example B). While less ethanol was needed in order to arrive at the desired viscosity for comparative composition C, comprising branched alkyl ethoxylated sulfate anionic surfactant, the composition did not provide the same level of initial sudsing or suds rinsing as inventive composition 1. In addition, since the alkyl glycol sulfate anionic surfactant used in inventive example 1 comprised 10 mol% or less of the sulfated anionic surfactant having two or more ethylene glycol units, the presence of 1,4-dioxane is substantially reduced in comparison to compositions comprising alkyl ethoxylated sulfate anionic surfactants, such as comparative examples B and C.

From the results of comparative examples D to G, it can be seen that increasing the ratio of anionic surfactant to the co-surfactant to 6:1 resulted in a significant reduction in the composition viscosity as well as a reduction in suds rinsing efficacy.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A liquid hand dishwashing detergent composition comprising from 5.0% to 50% by weight of the liquid hand dishwashing detergent composition of a surfactant system, wherein the surfactant system comprises:
a. anionic surfactant, wherein the anionic surfactant comprises alkyl glycol sulfate anionic surfactant, wherein the alkyl glycol sulfate anionic surfactant has the formula:
R1CH(R2)(OCH₂CH₂)ₙOSO3⁻M⁺ (I)
wherein:
R1 is independently H, alkyl, alkylene, or a mixture thereof;
R2 is independently alkyl, alkylene, or a mixture thereof;
the sum of the carbon atoms present in R1 and R2 is on average from 7 to 19;
n is from 1 to 3, wherein 90 mol% or greater of the surfactant molecules of structure (I) have an n of 1 and 10 mol% or less of the surfactant molecules of structure (I) have an n of 2 or greater; and
M+ is a counterion; and
b. co-surfactant selected from the group consisting of: amphoteric co-surfactant, zwitterionic co-surfactant, and mixtures thereof;
wherein the anionic surfactant and the co-surfactant are present in a weight ratio of from 1:1 to 5: 1.

2. The composition according to claim 1, wherein:
a. the composition comprises from 6.0% to 40%, preferably from 15% to 35%, by weight of the total composition of the surfactant system; and
b. the surfactant system comprises at least 40%, preferably from 60% to 90%, more preferably from 65% to 85% by weight of the surfactant system of the anionic surfactant.

3. The composition according to any of the preceding claims, wherein in the alkyl glycol sulfate anionic surfactant of formula (I), R1 comprises on average from 1 to 6 carbon atoms, preferably from 1 to 3 carbon atoms.

4. The composition according to claim 3, wherein in at least 80%, more preferably in at least 90% by mol of the alkyl glycol sulfate anionic surfactant of formula (I), R1 comprises 1 carbon atom, more preferably methyl.

5. The composition according to any of the preceding claims, wherein in the alkyl glycol sulfate anionic surfactant of formula (I), R2 comprises on average from 6 to 19 carbon atoms, preferably from 8 to 17 carbon atoms, more preferably from 9 to 15 carbon atoms, most preferably from 10 to 13 carbon atoms.

6. The composition according to any of the preceding claims, wherein in the alkyl glycol sulfate anionic surfactant of formula (I), R2 is linear, branched, or a combination thereof, preferably R2 is linear.

7. The composition according to any of the preceding claims, wherein in the alkyl glycol sulfate anionic surfactant of formula (I), the sum of the carbon atoms present in R1 and R2 is on average from 9 to 17, more preferably from 11 to 15, most preferably 11 to 13.

8. The composition according to any of the preceding claims, wherein in the alkyl glycol sulfate anionic surfactant of formula (I), 92 mol% or greater of the surfactant molecules of structure (I) have an n of 1 and 8 mol% or less of the surfactant molecules of structure (I) have an n of 2 or greater, preferably, 95 mol% or greater of the surfactant molecules of structure (I) have an n of 1 and 5 mol% or less of the surfactant molecules of structure (I) have an n of 2 or greater.

9. The composition according to any of the preceding claims, wherein the anionic surfactant further comprises alkyl sulfated anionic surfactant, more preferably wherein the anionic surfactant comprises at least 70%, more preferably at least 85%, most preferably 100% by weight of the anionic surfactant of the alkyl sulfated anionic surfactant and the alkyl glycol sulfate anionic surfactant.

10. The composition according to claim 9, wherein:
a. the alkyl sulfated anionic surfactant has a number average alkyl chain length of from 8 to 18, preferably from 10 to 14, more preferably from 12 to 14, most preferably from 12 to 13 carbon atoms;
b. the alkyl sulfated anionic surfactant has an average degree of alkoxylation of less than 1.0, preferably less than 0.5, more preferably less than 0.25, even more preferably less than 0.1, and most preferably wherein the alkyl sulfated anionic surfactant is free of alkoxylation;
c. the alkyl sulfated anionic surfactant has an average degree of branching of less than 60%, preferably less than 40%, more preferably less than 20%, most preferably less than 10%; and
d. combinations thereof.

11. The composition according to any of claims 9 to 10, wherein the alkyl sulfated anionic surfactant and the alkyl glycol sulfate anionic surfactant are present at a weight ratio of from 10:1 to 1:2, preferably from 7:1 to 1:1, and most preferably from 5:1 to 2:1.

12. The composition according to any preceding claim, wherein the anionic surfactant and the co-surfactant are present in a weight ratio of from 1.5:1 to 4.5:1, more preferably from 2:1 to 4:1,

13. The composition according to any preceding claim, wherein:
the amphoteric surfactant preferably is an amine oxide surfactant, more preferably wherein the amine oxide surfactant is selected from the group consisting of: alkyl dimethyl amine oxide, alkyl amido propyl dimethyl amine oxide, and mixtures thereof, most preferably alkyl dimethyl amine oxide; and
the zwitterionic surfactant preferably is a betaine surfactant, more preferably a betaine surfactant selected from the group consisting of alkyl betaines, alkylamidoalkylbetaine, amidazoliniumbetaine, sulfobetaine (INCI Sultaines), phosphobetaine, and mixtures thereof,
most preferably cocoamidopropylbetaine.

14. The composition according to any preceding claim, wherein the surfactant system further comprises nonionic surfactant.

15. The composition according to any preceding claim, wherein the composition has a pH of 7.0 or greater, preferably from 7.0 to 12.0, more preferably from 8.0 to 11.0, most preferably from 8.5 to 10.0, measured as a 10% aqueous solution in demineralized water at 20 degrees °C.
